(19) Europäisches Patentamt
European Patent Office
Office européen des brevets

(11) EP 1 368 474 B1

(12) **FASCICULE DE BREVET EUROPEEN**

(45) Date de publication et mention de la délivrance du brevet:
**17.01.2007 Bulletin 2007/03**

(21) Numéro de dépôt: **02748337.9**

(22) Date de dépôt: **01.03.2002**

(51) Int Cl.:
***C12N 15/12*** (2006.01) ***C07K 14/435*** (2006.01)
***C07K 16/18*** (2006.01) ***C12N 15/63*** (2006.01)
***A61K 38/17*** (2006.01) ***A61P 35/00*** (2006.01)

(86) Numéro de dépôt international:
**PCT/FR2002/000740**

(87) Numéro de publication internationale:
**WO 2002/070700 (12.09.2002 Gazette 2002/37)**

# (54) POLYNUCLEOTIDE UTILISABLE POUR MODULER LA PROLIFERATION DES CELLULES CANCEREUSES

ZUR MODULATION DER KREBSZELLENPROLIFERATION GEEIGNETES POLYNUKLEOTID

POLYNUCLEOTIDE USEFUL FOR MODULATING CANCER CELL PROLIFERATION

(84) Etats contractants désignés:
**AT BE CH CY DE DK ES FI FR GB GR IE IT LI LU MC NL PT SE TR**

(30) Priorité: **01.03.2001 FR 0102801**

(43) Date de publication de la demande:
**10.12.2003 Bulletin 2003/50**

(73) Titulaires:
- **Société de Conseils de Recherches et d'Applications Scientifiques ( S.C.R.A.S.)**
  **75016 Paris (FR)**
- **CENTRE NATIONAL DE LA RECHERCHE SCIENTIFIQUE (CNRS)**
  **75794 Paris Cedex 16 (FR)**

(72) Inventeurs:
- **FERRANDIS, Eric**
  **F-78470 Saint Remy Les Chevreuse (FR)**
- **CAMARA Y FERRER, José-Antonio**
  **F-75016 Paris (FR)**
- **MARTINEZ, Jean**
  **F-34720 Caux (FR)**
- **THURIEAU, Christophe**
  **F-75116 Paris (FR)**

(74) Mandataire: **Bourgouin, André**
**Beaufour Ipsen - S.C.R.A.S.,**
**24, rue Erlanger**
**75781 Paris Cedex 16 (FR)**

(56) Documents cités:
**WO-A-00/69454**

- **DATABASE EMBL [en ligne] HSM802477, 18 février 2000 (2000-02-18) Database accession no. AL157485 XP002186635**
- **DATABASE EMBL [en ligne] 22 février 2000 (2000-02-22) Database accession no. AK000755 XP002186636 cité dans la demande**
- **DATABASE EMBL [en ligne] HSM802912, 12 janvier 2001 (2001-01-12) Database accession no. AL512709 XP002186637**
- **FAN SAIJUN ET AL: "BRCA1 as a potential human prostate tumor suppressor: Modulation of proliferation, damage responses and expression of cell regulatory proteins." ONCOGENE, vol. 16, no. 23, 11 juin 1998 (1998-06-11), pages 3069-3082, XP001051838 ISSN: 0950-9232**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 368 474 B1

LISTE DE SEQUENCES

<110> Société de Conseils de Recherches et d'Application

<120> Nouveau polynucléotide utilisable pour moduler la prolifération des cellules cancéreuses

<130> RS 314 PCT - Hap-1

<140>
<141>

<150> FR 01/02801
<151> 2001-03-01

<160> 9

<170> PatentIn Ver. 2.1

<210> 1
<211> 576
<212> ADN
<213> Homo sapiens

<400> 1

```
ctccatgana tgccactgtt cctgaccagc cgaagtccag gcagggggag gtttccttgt 60
ccaggaggga gaggacctcc accctctgac ctttcagttt tccatagcag cccagtgtga 120
agctcggcag ngggtgggca gggcatcagg acaagatgaa gatgctggct ctggaatgag 180
gactcccctt ggcataaggg gtagtggctg gcagaggttg cccagctctt ttggaggcca 240
cagggaatag aggtggggtg gtgcttccgt tcctgtgagg cccgcccccc tgagcccccct 300
ccgtctgctt cttgtttggc agaaatagat gtggggcaca gcctaagaat gatgggaatc 360
tgcccaagct gtgccctctg gggaaatgaa atcttgctcc tcgtgcagct tcccctttttc 420
ccttgcgggg tgggggctgg gggtgccaag gctccaccct tggaaggggt tgctggggtg 480
ccaagctcca acctttgctc gctggcagaa ggaaggaagc acctcctgta tctttcaggg 540
ncctgaagcc antttgcctc agagaagaga nagtcc                           576
```

<210> 2

<211> 23
<212> ADN
<213> Séquence artificielle

<220>
<223> Description de la séquence artificielle: Oligonucléotide 5'

<400> 2
ctgacctttc agttttccat agc 23

<210> 3
<211> 22
<212> ADN
<213> Séquence artificielle

<220>
<223> Description de la séquence artificielle: Oligonucléotide 3'

<400> 3
atctatttct gccaaacaag aa 22

<210> 4
<211> 1009
<212> ADN
<213> Homo sapiens

<300>
<308> GenBank: AK000755

<400> 4

```
aggagggtct gaaggtggga gggcagctcc atgagcagcc actgttcctg accagccgaa 60
gtgccaggca gggggaggtt tccatgtcca ggagggagag gacctccacc ctctgacctt 120
tcagttttcc atagcagccc agtgtgaagc tcggcggggg tgggcagggc atcaggacaa 180
gatgaagatg ctggctctgg aatgaggact cccccttgggc taaggggtag tggctggcag 240
aggttgccca gctcttttgg aggccacagg gaatagaggt ggggtggtgc ttccgttcct 300
gtgaggcccg cccccctgag ccccctcgtc tgcttcttgt ttggcagaaa tagatgtggg 360
gcacagcccc tagatgatgg gaatctgccc aagctgtgtc ctctggggaa tgaaatcttg 420
ctcctcatgc agcttcccct ttcccttgcg gggtgggggc tgggggtgcc aaggctccac 480
ccttggaggg ggttgctggg ggtgccaagg ctccaccctt tgctcgctgg gcagagggaa 540
ggaagcacct ccctgtatgc tctgcagggg cccctgcagg ccactttgcc tcagaggaag 600
gaggagaggt cccctgggaa aatgcgcaca cacacacaca cacacacacg cacacacaca 660
cgcacatgca cacacgcacg catgcacaca cacacacata tgtgcagggg tgcagcctaa 720
gtcagtggag ccaggactgg ctgcgcagct gggcctgccc cattcttgga gtccccaggg 780
aatgagcctc aggatccact tcgtcccttg tttctaagcc aggctgcacg gccgttcctg 840
ggtgtgaaag cattctgtcc tgtctccagt cagagggaac cgccccccaaa ctagaaagct 900
aatttccccc gaaggttcat gaagccagag gcatgccctt gggggttggt cgggagaagg 960
gtgagaggca gtgctgtgga aggggcctgg cccctgctgc ctgcgattc 1009
```

<210> 5
<211> 1055
<212> ADN

<213> Homo sapiens

<400> 5

```
aggagggtct gaaggtggga gggcagctcc atgagcagcc actgttcctg accagccgaa 60
gtgccaggca gggggaggtt tccatgtcca ggagggagag gacctccacc ctctgacctt 120
tcagttttcc atagcagccc agtgtgaagc tcggcggggg tgggcagggc atcaggacaa 180
gatgaagatg ctggctctgg aatgaggact ccccttgggc taaggggtag tggctggcag 240
aggttgccca gctcttttgg aggccacagg gaatagaggt ggggtggcaa caggaaatag 300
ggtgtgggct gtagaccata gtgggtgggg tggtgcttcc gttcctgtga ggcccgcccc 360
cctgagcccc ctcgtctgct tcttgtttgg cagaaataga tgtggggcac agcccctaga 420
tgatgggaat ctgcccaagc tgtgtcctct ggggaatgaa atcttgctcc tcatgcagct 480
tcccctttcc cttgcggggt gggggctggg ggtgccaagg ctccaccctt ggaggggggtt 540
gctgggggtg ccaaggctcc accctttgct cgctgggcag agggaaggaa gcacctccct 600
gtatgctctg caggggcccc tgcaggccac tttgcctcag aggaaggagg agaggtcccc 660
tgggaaaatg cgcacacaca cacacacaca cacacgcaca cacacacgca catgcacaca 720
cgcacgcatg cacacacaca cacatatgtg caggggtgca gcctaagtca gtggagccag 780
gactggctgc gcagctgggc ctgccccatt cttggagtcc ccagggaatg agcctcagga 840
tccacttcgt cccttgtttc taagccaggc tgcacggccg ttcctgggtg tgaaagcatt 900
ctgtcctgtc tccagtcaga gggaaccgcc cccaaactag aaagctaatt tcccccgaag 960
gttcatgaag ccagaggcat gcccttgggg gttggtcggg agaagggtga gaggcagtgc 1020
tgtggaaggg gcctggcccc tgctgcctgc gattc                            1055
```

<210> 6
<211> 25
<212> ADN
<213> Séquence artificielle

<220>
<223> Description de la séquence artificielle: Oligonucléotide 5'

<400> 6
gcaagcttgc gggggaggag gaggg 25

<210> 7
<211> 24
<212> ADN
<213> Séquence artificielle

<220>
<223> Description de la séquence artificielle: Oligonucléotide 3'

<400> 7
cggaattccc ggggggaatc gcag 24

<210> 8
<211> 147
<212> PRT
<213> Homo sapiens

<400> 8

```
Met Met Gly Ile Cys Pro Ser Cys Val Leu Trp Gly Met Lys Ser Cys
1               5                   10                  15

Ser Ser Cys Ser Phe Pro Phe Pro Leu Arg Gly Gly Gly Trp Gly Cys
            20                  25                  30

Gln Gly Ser Thr Leu Gly Gly Gly Cys Trp Gly Cys Gln Gly Ser Thr
        35                  40                  45

Leu Cys Ser Leu Gly Arg Gly Lys Glu Ala Pro Pro Cys Met Leu Cys
    50                  55                  60

Arg Gly Pro Cys Arg Pro Leu Cys Leu Arg Gly Arg Arg Arg Gly Pro
65                  70                  75                  80

Leu Gly Lys Cys Ala His Thr His Thr His Thr His Ala His Thr His
                85                  90                  95

Ala His Ala His Thr His Ala Cys Thr His Thr His Ile Cys Ala Gly
            100                 105                 110

Val Gln Pro Lys Ser Val Glu Pro Gly Leu Ala Ala Gln Leu Gly Leu
        115                 120                 125

Pro His Ser Trp Ser Pro Gln Gly Met Ser Leu Arg Ile His Phe Val
    130                 135                 140

Pro Cys Phe
145
```

<210> 9
<211> 444
<212> ADN
<213> Homo sapiens

<400> 9

```
atgatgggaa tctgcccaag ctgtgtcctc tggggaatga aatcttgctc ctcatgcagc 60
ttcccctttc ccttgcgggg tgggggctgg gggtgccaag gctccaccct tggagggggt 120
tgctgggggt gccaaggctc caccctttgc tcgctgggca gagggaagga agcacctccc 180
tgtatgctct gcaggggccc ctgcaggcca ctttgcctca gaggaaggag gagaggtccc 240
ctgggaaaat gcgcacacac acacacacac acacacgcac acacacacgc acatgcacac 300
acgcacgcat gcacacacac acacatatgt gcaggggtgc agcctaagtc agtggagcca 360
```

```
ggactggctg cgcagctggg cctgccccat tcttggagtc cccagggaat gagcctcagg 420
atccacttcg tcccttgttt ctaa                                        444
```

## Description

La présente invention concerne une nouvelle protéine modulant la prolifération des cellules cancéreuses. Grâce aux protéines codées par les polynucléotides de séquences SEQ. ID NO. 4, SEQ. ID. NO. 5 et SEQ. ID. NO. 9 décrites plus loin, il est également possible de déterminer le degré de malignité de cellules en prolifération anormale.

Le cancer est un problème de santé majeur dans le monde. Bien que des progrès importants aient été réalisés ces dernières années dans la détection et le traitement du cancer, aucun vaccin ou autre méthode de traitement universel pour la prévention ou le traitement du cancer n'existe actuellement. La gestion de la maladie réside dans la combinaison d'un diagnostic aussi précoce que possible et d'un traitement agressif incluant une variété d'éléments comme la chirurgie, la radiothérapie, la chimiothérapie ou encore l'hormonothérapie. L'évolution du traitement pour un cancer donné est souvent sélectionnée sur la base de paramètres pronostics comprenant une analyse de marqueurs spécifiques de tumeurs. Cependant, l'utilisation de marqueurs établis conduit souvent à un résultat difficilement interprétable et une mortalité accrue continue d'être observée chez de nombreux patients cancéreux.

Les cancers du sein et de la prostate sont les tumeurs malignes les plus fréquentes. Aux Etats-Unis, 400 000 nouveaux cas sont diagnostiqués chaque année et environ 100 000 hommes et femmes meurent chaque année de leur maladie (Harris et coll., *New Eng. J. Med.* (1992), **327**, 319, 390 et 473).

Il est admis que ces maladies apparaissent lors d'un processus multi-factoriel impliquant des mutations dans un nombre restreint de gènes, peut-être 10 ou moins. Ces mutations entraînent un changement de croissance et de modulation de la prolifération cellulaire des tissus leur permettant de croître indépendamment des contrôles cellulaires normaux, de former des métastases et d'échapper à la surveillance immunitaire. Les classes de gènes impliqués dans les cancers du sein et de la prostate peuvent être hautement spécifiques de ces classes de tumeurs. En particulier, les mutations dans les gènes impliqués dans les réponses hormonales (récepteurs et ligands de la classe des oestrogènes, de la progestérone ou bien encore de la testostérone) sont particulièrement importantes car elles poussent probablement les cellules à proliférer tout en les rendant réfractaires à l'effet anti-tumoral de ces hormones.

De plus, des changements dans les gènes codant pour des facteurs de croissance, des molécules de transduction du signal ainsi que des facteurs de transcription sont fréquemment impliqués et ont des altérations qui sont elles-mêmes impliquées dans le développement et la progression tumorale (King, *Nature Genetics* (1992), **2**,125).

Une question non résolue à ce jour est la nature des changements de l'expression génique qui apparaît dans les cellules tumorales humaines de manière irréversible conduisant à la différenciation cellulaire. Cette information est pourtant très importante pour la définition, sur le plan moléculaire, des schémas de régulation de l'expression génique impliqués dans la croissance et la différenciation cellulaire des cellules humaines de cancers de la prostate et du sein. Il existe ainsi un besoin urgent d'identification de séquences géniques impliquées dans le rendez-vous irréversible de la différenciation terminale.

Tout récemment, alors que la demanderesse était déjà en possession de l'invention décrite ci-après, la séquence SEQ. ID. NO. 4 a été divulguée dans la base de données Genbank sous le numéro d'accès AK000755 et le numéro d'identification 7021039, sans indication toutefois de l'activité des protéines pouvant être codées par cette séquence.

La présente invention a pour objet un polynucléotide isolé comprenant la séquence polynucléotidique SEQ. ID. NO. 5. Elle a aussi pour objet un polynucléotide anti-sens comprenant la séquence complémentaire à celle dudit polynucléotide isolé comprenant la séquence polynucléotidique SEQ. ID. NO. 5.

L'invention concerne aussi un polynucléotide isolé comprenant au moins un fragment de la séquence polynucléotidique SEQ. ID. NO. 5, ledit polynucléotide étant tel qu'il code pour un polypeptide ayant au moins une activité immunologique et/ou biologique caractéristique d'une protéine associée à la modulation de la prolifération cellulaire. En particulier, l'invention concerne à ce titre le polynucléotide isolé de séquence nucléotidique SEQ. ID. NO. 9 ou le polynucléotide isolé de séquence nucléotidique complémentaire à la séquence nucléotidique SEQ. ID. NO. 9.

L'invention a de plus pour objet un polypeptide isolé comprenant au moins un fragment de la protéine codée par la séquence polynucléotidique SEQ. ID. NO. 5 ou par une séquence complémentaire de la séquence polynucléotidique SEQ. ID. NO. 5, ledit polypeptide isolé ayant au moins une activité immunologique et/ou biologique caractéristique d'une protéine associée à la modulation de la prolifération cellulaire. De préférence, ledit polypeptide isolé possède un fragment ayant une activité immunologique caractéristique d'une protéine associée à la modulation de la prolifération cellulaire.

En particulier, l'invention concerne la protéine de séquence SEQ. ID. NO. 8 (décrite plus loin) codée par le fragment du polynucléotide de séquence polynucléotidique SEQ. ID. NO. 5 contenu entre les bases aux positions 420 (codon initiateur ATG codant pour une méthionine) et 861 (codon stop UAA), i.e. par la séquence polynucléotidique SEQ. ID. NO. 9 (décrite plus loin).

L'invention concerne encore un vecteur d'expression comprenant un polynucléotide isolé comprenant au moins un fragment de la séquence polynucléotidique SEQ. ID. NO. 5 ou de la séquence complémentaire de la séquence polynucléotidique SEQ. ID. NO. 5, le polypeptide codé par ledit polynucléotide isolé ayant au moins une activité immunologique et/ou biologique caractéristique d'une protéine associée à la modulation de la prolifération cellulaire. Elle concerne de même une cellule hôte transformée ou transfectée avec ledit vecteur d'expression.

L'invention a également pour objet un anticorps monoclonal, ou un fragment de liaison de l'antigène de celui-ci, qui fixe spécifiquement un polypeptide isolé tel que décrit précédemment, et en particulier un anticorps monoclonal, ou un fragment de liaison de l'antigène de celui-ci, qui fixe spécifiquement la protéine de séquence SEQ. ID. NO. 8.

L'invention concerne de plus, à titre de médicament, un polypeptide isolé comprenant au moins un fragment de la protéine codée par la séquence polynucléotidique SEQ. ID. NO. 5 ou par une séquence complémentaire de la séquence polynucléotidique SEQ. ID. NO. 5, ledit polypeptide isolé ayant au moins une activité immunologique et/ou biologique caractéristique d'une protéine associée à la modulation de la prolifération cellulaire. En particulier, ledit fragment peut être le fragment codé par le polynucléotide de séquence nucléotidique SEQ. ID. NO. 9 ou par la séquence complémentaire de la séquence polynucléotidique SEQ. ID. NO. 9, autrement dit la protéine de séquence SEQ. ID. NO. 8. Elle concerne de même, à titre de médicament, un polypeptide isolé comprenant au moins un fragment de la protéine codée par la séquence polynucléotidique SEQ. ID. NO. 4 ou par une séquence complémentaire de la séquence polynucléotidique SEQ. ID. NO. 4, ledit polypeptide isolé ayant au moins une activité immunologique et/ou biologique caractéristique d'une protéine associée à la modulation de la prolifération cellulaire.

L'invention concerne encore, à titre de médicament, un anticorps monoclonal, ou un fragment de liaison de l'antigène de celui-ci, qui fixe spécifiquement un polypeptide isolé comprenant au moins un fragment de la protéine codée par la séquence polynucléotidique SEQ. ID. NO. 4 ou SEQ. ID. NO. 5 ou par une séquence complémentaire de la séquence polynucléotidique SEQ. ID. NO. 4 ou SEQ. ID. NO. 5, ledit polypeptide isolé ayant au moins une activité immunologique et/ou biologique caractéristique d'une protéine associée à la modulation de la prolifération cellulaire.

En particulier, l'invention concerne, à titre de médicament, un anticorps monoclonal, ou un fragment de liaison de l'antigène de celui-ci, qui fixe spécifiquement un polypeptide isolé codé par le polynucléotide de séquence nucléotidique SEQ. ID. NO. 9 ou par la séquence complémentaire de la séquence polynucléotidique SEQ. ID. NO. 9 (autrement dit la protéine de séquence SEQ. ID. NO. 8).

L'invention concerne par ailleurs une composition pharmaceutique comprenant un polypeptide isolé comprenant :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la prolifération cellulaire;
ladite composition comprenant par ailleurs un ou des excipients pharmaceutiquement acceptables.

En particulier, l'invention aura pour objet une composition pharmaceutique comprenant la protéine codée par la séquence polynucléotidique SEQ. ID. NO. 9 ou par la séquence complémentaire de la séquence polynucléotidique SEQ. ID. NO. 9 (autrement dit la protéine de séquence SEQ. ID. NO. 8) avec un ou des excipients pharmaceutiquement acceptables.

Alternativement, une composition pharmaceutique selon l'invention comprendra un anticorps monoclonal, ou un fragment de liaison de l'antigène de celui-ci, qui fixe spécifiquement un polypeptide isolé comprenant au moins un fragment de la protéine codée par la séquence polynucléotidique SEQ. ID. NO. 4 ou SEQ. ID. NO. 5 ou par une séquence complémentaire de la séquence polynucléotidique SEQ. ID. NO. 4 ou SEQ. ID. NO. 5, ledit polypeptide isolé ayant au moins une activité immunologique et/ou biologique caractéristique d'une protéine associée à la modulation de la prolifération cellulaire, ladite composition comprenant par ailleurs un ou des excipients pharmaceutiquement acceptables.

En particulier, l'invention concerne une composition pharmaceutique comprenant un anticorps monoclonal, ou un fragment de liaison de l'antigène de celui-ci, qui fixe spécifiquement la protéine codée par la séquence polynucléotidique SEQ. ID. NO. 9 ou par la séquence complémentaire de la séquence polynucléotidique SEQ. ID. NO. 9 (autrement dit la protéine de séquence SEQ. ID. NO. 8) avec un ou des excipients pharmaceutiquement acceptables.

Selon une variante préférée, lesdites compositions pharmaceutiques comprendront également un activateur de la réponse immunitaire (lequel peut être un adjuvant).

Un objet supplémentaire de l'invention est l'utilisation, pour préparer un médicament destiné à traiter une maladie proliférative, d'un polypeptide isolé comprenant :

- soit un fragment de la protéine codée par la séquence polynucléotidique SEQ. ID. NO. 5 ou par une séquence complémentaire de la séquence polynucléotidique SEQ. ID. NO. 5,

- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la prolifération cellulaire.

En particulier, l'invention concerne l'utilisation, pour préparer un médicament destiné à traiter une maladie proliférative, d'un polypeptide isolé codé par la séquence polynucléotidique SEQ. ID. NO. 9 ou par la séquence complémentaire de la séquence polynucléotidique SEQ. ID. NO. 9 (autrement dit, de la protéine de séquence SEQ. ID. NO. 8) avec un ou des excipients pharmaceutiquement acceptables.

Un autre objet de l'invention est l'utilisation d'un polynucléotide isolé comprenant :

- la séquence polynucléotidique SEQ. ID. NO. 5, ou
- la séquence polynucléotidique SEQ. ID. NO. 4, ou encore
- la séquence polynucléotidique SEQ. ID. NO. 9,

pour préparer un médicament destiné à traiter une maladie proliférative.

De préférence, le polynucléotide isolé utilisé consistera en un polynucléotide de séquence polynucléotidique SEQ. ID. NO. 4, SEQ. ID. NO. 5 ou SEQ. ID. NO. 9.

Ledit polynucléotide isolé utilisé est de préférence présent dans un vecteur viral, ledit vecteur viral étant par exemple sélectionné à partir du groupe consistant en un adénovirus, un adénovirus associé, un rétrovirus et un pox virus.

Alternativement, toujours selon la présente invention, un anticorps monoclonal, ou un fragment de liaison de l'antigène de celui-ci, qui fixe spécifiquement un polypeptide isolé comprenant au moins un fragment de la protéine codée par la séquence polynucléotidique SEQ. ID. NO. 4 ou SEQ. ID. NO. 5 ou par une séquence complémentaire de la séquence polynucléotidique SEQ. ID. NO. 4 ou SEQ. ID. NO. 5, ledit polypeptide isolé ayant au moins une activité immunologique et/ou biologique caractéristique d'une protéine associée à la modulation de la prolifération cellulaire, pourra être utilisé pour préparer un médicament destiné à traiter une maladie proliférative.

En particulier, un anticorps monoclonal, ou un fragment de liaison de l'antigène de celui-ci, qui fixe spécifiquement un polypeptide isolé codé par la séquence polynucléotidique SEQ. ID. NO. 9 ou par la séquence complémentaire à la séquence polynucléotidique SEQ. ID. NO. 9 (autrement dit, un anticorps monoclonal, ou un fragment de liaison de l'antigène de celui-ci, qui fixe spécifiquement la protéine de séquence SEQ. ID. NO. 8), pourra être utilisé pour préparer un médicament destiné à traiter une maladie proliférative.

Selon des variantes préférées des utilisations susmentionnées, la maladie proliférative à traiter par le polypeptide ou le polynucléotide décrits précédemment sera un cancer. Selon des variantes encore plus préférées, le cancer sera choisi parmi le groupe consistant en le cancer de la prostate, le cancer du sein, le cancer du poumon et le cancer colorectal.

L'invention offre de plus une méthode pour déterminer si une tumeur chez un patient est maligne, ladite méthode comprenant la détermination, dans un échantillon tumoral obtenu d'un patient, de la concentration en un polypeptide isolé comprenant au moins :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la prolifération cellulaire.

En particulier, ladite méthode de détermination pourra avoir pour objet la détermination, dans un échantillon tumoral obtenu d'un patient, de la concentration en polypeptide isolé codé par la séquence polynucléotidique SEQ. ID. NO. 9 ou par la séquence complémentaire à la séquence polynucléotidique SEQ. ID. NO. 9 (autrement dit, de la concentration en protéine de séquence SEQ. ID. NO. 8).

Selon une variante préférée de la méthode susmentionnée, ladite méthode comprend la mise en contact de l'échantillon tumoral avec un anticorps monoclonal qui reconnaît spécifiquement le polypeptide isolé précédemment mentionné (et en particulier polypeptide isolé codé par la séquence polynucléotidique SEQ. ID. NO. 9 ou par la séquence complémentaire à la séquence polynucléotidique SEQ. ID. NO. 9 (autrement dit, de la concentration en protéine de séquence SEQ. ID. NO. 8)).

L'invention concerne aussi une méthode pour déterminer si une tumeur chez un patient est maligne, ladite méthode comprenant la détermination, dans un échantillon biologique obtenu d'un patient, de la concentration :

- en polynucléotide de séquence polynucléotidique SEQ. ID. NO. 5 ou SEQ. ID. NO. 4, ou
- en polynucléotide dont la séquence nucléotidique est un fragment de la séquence polynucléotidique SEQ. ID. NO. 5 ou SEQ. ID. NO. 4, ledit fragment codant pour un polypeptide isolé ayant au moins une activité immunologique et/ou biologique caractéristique d'une protéine associée à la modulation de la prolifération cellulaire.

En particulier, ladite méthode comprendra la détermination, dans un échantillon biologique obtenu d'un patient, de la concentration en polypeptide isolé codé par la séquence polynucléotidique SEQ. ID. NO. 9 ou par la séquence complémentaire à la séquence polynucléotidique SEQ. ID. NO. 9 (autrement dit, de la concentration en protéine de séquence SEQ. ID. NO. 8).

Selon une variante préférée de cette dernière méthode, ladite méthode comprend :

(a) la préparation de molécules d'ADNc à partir de molécules d'ARN de l'échantillon tumoral, puis

(b) l'amplification spécifique de molécules d'ADNc qui sont capables de coder pour au moins une portion du polypeptide.

L'invention offre encore une méthode pour suivre la progression ou la régression de la maladie chez un patient atteint d'un cancer, ladite méthode comprenant :

(a) la détermination, répétée à intervalles choisis au cours du temps, dans un échantillon biologique obtenu d'un patient, de la concentration en polypeptide isolé comprenant au moins :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la prolifération cellulaire, et

(b) la comparaison, au cours du temps, des concentrations déterminées en (a).

En particulier, ladite méthode pour suivre la progression ou la régression de la maladie chez un patient atteint d'un cancer comprendra, dans son étape (a), la détermination répétée à intervalles choisis au cours du temps, dans un échantillon biologique obtenu d'un patient, de la concentration en polypeptide isolé codé par la séquence polynucléotidique SEQ. ID. NO. 9 ou par la séquence complémentaire à la séquence polynucléotidique SEQ. ID. NO. 9 (autrement dit, de la concentration en protéine de séquence SEQ. ID. NO. 8).

L'étape (a) de la méthode ci-dessus comprendra de préférence la mise en contact d'une portion de l'échantillon biologique avec un anticorps monoclonal qui reconnaît spécifiquement ledit polypeptide (en particulier la protéine de séquence SEQ. ID. NO. 8). Selon une variante préférée de la méthode ci-dessus, l'échantillon biologique obtenu du patient sera par ailleurs une portion de tumeur.

Toujours selon l'invention, une autre méthode pour suivre la progression ou la régression de la maladie chez un patient atteint d'un cancer comprendra les étapes suivantes :

(a) la détermination, répétée à intervalles choisis au cours du temps, dans un échantillon biologique obtenu d'un patient, de la concentration en ARN codant pour un polypeptide isolé comprenant au moins :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la prolifération cellulaire, et

(b) la comparaison, au cours du temps, des concentrations déterminées en (a).

En particulier, ladite méthode pour suivre la progression ou la régression de la maladie chez un patient atteint d'un cancer comprendra, dans son étape (a), la détermination répétée à intervalles choisis au cours du temps, dans un échantillon biologique obtenu d'un patient, de la concentration en ARN codant pour un polypeptide isolé codé par la séquence polynucléotidique SEQ. ID. NO. 9 ou par la séquence complémentaire à la séquence polynucléotidique SEQ. ID. NO. 9 (autrement dit, de la concentration en ARN codant pour la protéine de séquence SEQ. ID. NO. 8).
Selon une variante préférée de la méthode de suivi décrite ci-dessus, l'étape (a) comprendra de préférence :

(i) la préparation de molécules d'ADNc à partir de molécules d'ARN dans l'échantillon biologique, et

(ii) l'amplification spécifique de molécules d'ADNc qui sont capables de coder au moins pour une portion dudit polypeptide isolé.

Toutes les méthodes de détermination et/ou de suivi décrites ci-dessus sont des outils qui permettront au médecin, après analyse des résultats, de formuler son diagnostic quant à la gravité et/ou la progression ou la régression des tumeurs du patient concerné.
L'invention a de plus pour objet un kit pour le diagnostic de maladies prolifératives comprenant :

(1) un anticorps monoclonal qui fixe spécifiquement un polypeptide isolé comprenant au moins un fragment de la protéine codée par la séquence polynucléotidique SEQ. ID. NO. 5 ou SEQ. ID. NO. 4, ou par une séquence complémentaire de la séquence polynucléotidique SEQ. ID. NO. 5 ou SEQ. ID. NO. 4,

(2) un second anticorps monoclonal ou un fragment de celui-ci qui se fixe soit à un anticorps monoclonal tel que défini en (1), soit au polypeptide isolé tel que défini en (1),

ledit second anticorps monoclonal étant conjugué à un groupement révélateur.
En particulier, ledit kit pour le diagnostic de maladies prolifératives pourra être choisi tel que le fragment de la protéine codée par la séquence polynucléotidique SEQ. ID. NO. 5 soit le polypeptide isolé codé par la séquence polynucléotidique SEQ. ID. NO. 9 ou par la séquence complémentaire de la séquence polynucléotidique SEQ. TD. NO.9 (autrement dit, la protéine de séquence SEQ. ID. NO. 8).
De préférence, le groupement révélateur du kit de diagnostic ci-dessus est sélectionné dans le groupe composé des radio-isotopes, des groupements fluorescents, des groupements luminescents, des enzymes, de la biotine et des particules colorées.
L'invention offre de plus une méthode de préparation d'un polypeptide isolé comprenant au moins un fragment de la protéine codée par la séquence polynucléotidique SEQ. ID. NO. 5 ou par une séquence complémentaire de la séquence polynucléotidique SEQ. ID. NO. 5, ledit polypeptide isolé ayant au moins une activité immunologique et/ou biologique caractéristique d'une protéine associée à la modulation de la prolifération cellulaire, ladite méthode de préparation comprenant les étapes successives suivantes :

(a) culture, dans des conditions convenables pour obtenir l'expression dudit polypeptide d'une cellule hôte transformée ou transfectée avec un vecteur d'expression comprenant un polynucléotide isolé comprenant au moins un fragment de la protéine codée par la séquence polynucléotidique SEQ. ID. NO. 5 ou par une séquence complémentaire de la séquence polynucléotidique SEQ. ID. NO. 5, ledit polypeptide isolé ayant au moins une activité immunologique et/ou biologique caractéristique d'une protéine associée à la modulation de la prolifération cellulaire, et

(b) isolement du polypeptide à partir des cultures de cellules hôtes.

En particulier, ladite méthode aura pour objet la préparation du polypeptide isolé codé par la séquence polynucléotidique SEQ. ID. NO. 9 ou par la séquence complémentaire de la séquence polynucléotidique SEQ. ID. NO. 9 (autrement dit, la préparation de la protéine de séquence SEQ. ID. NO. 8).
La présente invention offre encore une méthode pour l'identification de composés susceptibles de moduler la croissance et/ou la différenciation cellulaire, laquelle comprend les étapes successives suivantes :

(a) mise en contact de chaque composé candidat, dans des conditions et pendant un temps suffisant pour permettre à l'agent candidat de se fixer au polypeptide, avec un polypeptide isolé comprenant au moins :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la croissance et/ou la différenciation cellulaire, et

(b) détection de la fixation de chaque composé candidat audit polypeptide et identification, parmi les composés candidats, des composés susceptibles de moduler la croissance et/ou la différenciation cellulaire.

En particulier, ladite méthode pour l'identification de composés susceptibles de moduler la croissance et/ou la différenciation cellulaire comprendra, dans son étape (a), la mise en contact de chaque composé candidat, dans des conditions et pendant un temps suffisant pour permettre à l'agent candidat de se fixer au polypeptide, avec le polypeptide isolé codé par la séquence polynucléotidique SEQ. ID. NO. 9 ou par la séquence complémentaire de la séquence polynucléotidique SEQ. ID. NO. 9 (autrement dit, avec la protéine de séquence SEQ. ID. NO. 8).

Une méthode alternative pour l'identification de composés susceptibles de moduler la croissance et/ou la différenciation cellulaire comprend les étapes successives suivantes :

(a) mise en contact de chaque composé candidat, dans des conditions et pendant un temps suffisant pour permettre à l'agent candidat et à la cellule d'interagir, avec une cellule capable d'exprimer un polypeptide isolé comprenant au moins :

- soit un fragment de la protéine codée par la séquence polynucléotidique SEQ. ID. NO. 5 ou par une séquence complémentaire de la séquence polynucléotidique SEQ. ID. NO. 5,
- soit un fragment de la protéine codée par la séquence polynucléotidique SEQ. ID. NO. 4 ou par une séquence complémentaire de la séquence polynucléotidique SEQ. ID. NO. 4,

ledit polypeptide isolé ayant au moins une activité immunologique et/ou biologique caractéristique d'une protéine associée à la modulation de la croissance et/ou la différenciation cellulaire, et

(b) détermination de l'effet de chaque composé candidat sur la concentration cellulaire en polypeptide et identification, parmi les composés candidats, des composés susceptibles de moduler la croissance et/ou la différenciation cellulaire.

En particulier, ladite méthode alternative comprendra, dans son étape (a), la mise en contact de chaque composé candidat, dans des conditions et pendant un temps suffisant pour permettre à l'agent candidat et à la cellule d'interagir, avec une cellule capable d'exprimer polypeptide isolé codé par la séquence polynucléotidique SEQ. ID. NO. 9 ou par la séquence complémentaire de la séquence polynucléotidique SEQ. ID. NO. 9 (autrement dit, une cellule capable d'exprimer la protéine de séquence SEQ. ID. NO. 8).

Selon des modes d'exécution préférés des méthodes d'identification de composés susceptibles de moduler la croissance et/ou la différenciation décrites ci-dessus, les composés candidats proviendront de librairies de petites molécules issues de programmes de chimie combinatoire.

L'invention concerne de plus un polynucléotide comprenant un promoteur endogène ou un élément de régulation de la protéine associée à la différenciation, dans laquelle la protéine comprend une séquence codée par une séquence polynucléotidique SEQ. ID. NO. 5 ou par une séquence complémentaire de la séquence polynucléotidique SEQ. ID. NO. 5.

L'invention concerne également un polynucléotide comprenant un gène de révélation sous le contrôle du promoteur endogène ou des éléments de régulation de la protéine associée à la modulation de la prolifération cellulaire, dans laquelle la protéine comprend une séquence codée par la séquence polynucléotidique SEQ. ID. NO. 5 ou par une séquence complémentaire de la séquence polynucléotidique SEQ. ID. NO. 5. L'invention concerne de la même façon une cellule transformée ou transfectée avec ce dernier polynucléotide.

L'invention concerne par ailleurs une méthode pour identifier des composés susceptibles de moduler l'expression de la protéine associée à la modulation de la prolifération cellulaire, laquelle comprend les étapes successives suivantes :

(a) mise en contact de chaque composé candidat, dans des conditions telles et pendant un temps suffisant pour permettre à chaque composé candidat d'interagir avec le promoteur ou l'élément de régulation de celui-ci, avec une cellule transformée ou transfectée avec un polynucléotide comprenant un gène de révélation sous le contrôle du promoteur endogène ou des éléments de régulation de la protéine associée à la modulation de la prolifération cellulaire, dans laquelle la protéine comprend une séquence codée par la séquence polynucléotidique SEQ. ID. NO. 5 ou par une séquence complémentaire de la séquence polynucléotidique SEQ. ID. NO. 5,

(b) détermination des effets de chaque composé candidat sur la concentration en protéine de révélation produite par la cellule, et identification des composés susceptibles de moduler l'expression de la protéine associée à la modulation de la prolifération cellulaire.

Selon un mode d'exécution préféré de la méthode d'identification de composés susceptibles de moduler l'expression de la protéine associée à la modulation de la prolifération cellulaire décrite ci-dessus, les composés candidats proviendront de librairies de petites molécules issues de programmes de chimie combinatoire.
Les propriétés pharmacologiques obtenues pour les polynucléotides et polypeptides selon l'invention rendent ces derniers aptes à une utilisation pharmaceutique. En effet, les polypeptides isolés comprenant au moins:

- soit un fragment de la protéine codée par la séquence polynucléotidique SEQ. ID. NO. 5 ou par une séquence complémentaire de la séquence polynucléotidique SEQ. ID. NO. 5,

- soit un fragment de la protéine codée par la séquence polynucléotidique SEQ. ID. NO. 4 ou par une séquence complémentaire de la séquence polynucléotidique SEQ. ID. NO. 4,

qui ont au moins une activité immunologique et/ou biologique caractéristique d'une protéine associée à la modulation de la prolifération cellulaire, ainsi que les polynucléotides codant pour lesdits polypeptides, peuvent, selon l'invention, être administrés à des patients cancéreux afin de ralentir la progression de leurs tumeurs ou de faire régresser lesdites tumeurs.
Dans les méthodes ci-dessus, la protéine associée à la modulation de la prolifération cellulaire ou à la différenciation pourra en particulier être le polypeptide isolé codé par la séquence polynucléotidique SEQ. ID. NO. 9 ou par la séquence complémentaire de la séquence polynucléotidique SEQ. ID. NO. 9 (autrement dit, la protéine de séquence SEQ. ID. NO. 8).
Les différents éléments évoqués ci-dessus deviendront évidents pour l'homme du métier une fois faite la lecture de la description plus détaillée des différents aspects de l'invention.

## Description détaillée des différents aspects de l'invention

Comme mentionné ci-dessus, la présente invention est généralement dirigée vers des produits et des méthodes destinés à moduler la croissance cellulaire et à traiter le cancer. La présente invention est basée, en partie, sur l'identification de « séquences associées à la modulation de la prolifération cellulaire » qui sont des séquences polypeptidiques et polynucléotidiques associées à la modulation de la prolifération cellulaire. Un ARNm associé à la différenciation est un ARNm qui est présent à un niveau plus élevé dans des cellules différenciées que dans les cellules correspondantes non différenciées (i.e. le niveau d'ARNm est au moins 2 fois plus élevé). Une molécule d'ADNc associée à la différenciation est une séquence correspondant à un ARNm associé à la différenciation (et/ou une séquence complémentaire).
De telles molécules d'ADNc peuvent être préparées à partir de préparations d'ARN ou d'ARNm en utilisant les techniques standard, comme la transcription inverse. De manière similaire, une protéine ou un polypeptide associé à la différenciation comprend la séquence codée par un ARNm associé à la différenciation cellulaire.
Les compositions pharmaceutiques décrites ici peuvent inclure un ou plusieurs polypeptides, séquences d'acides nucléiques et/ou anticorps. Les polypeptides de la présente invention comprennent au moins une portion de la protéine associée à la modulation de la prolifération cellulaire ou un variant de celle-ci. Les séquences d'acides nucléiques de la présente invention comprennent une séquence d'ADN ou d'ARN qui code au moins pour une portion d'un tel polypeptide ou qui est complémentaire à une telle séquence codante.
Les anticorps sont des protéines du système immunitaire ou des fragments de fixation à l'antigène de celui-ci, qui sont capables de fixer une portion des polypeptides décrits ci-dessus.
Alternativement, une composition peut comprendre un ou plusieurs agents qui modulent l'expression du gène associé à la modulation de la prolifération cellulaire.

Polynucléotides, polypeptides et protéines selon l'invention :

La présente invention a en particulier pour objet les polynucléotides de séquence SEQ. ID. NO. 4, SEQ. ID. NO. 5 ou SEQ. ID. NO. 9 ainsi que le polypeptide ou la protéine de séquence SEQ. ID. NO. 8.

L'invention comprend également les polynucléotides possédant des séquences polynucléotidiques homologues au moins à 75 %, de préférence au moins à 85 % et encore plus préférentiellement au moins à 90 % voire 95 %, aux séquences des polynucléotides décrits plus haut, notamment aux séquences SEQ. ID. NO. 4, SEQ. ID. NO. 5 et SEQ. ID. NO. 9. Cela s'applique également *mutatis mutandis* aux autres polynucléotides, polypeptides et protéines faisant partie de l'invention, notamment à la protéine de séquence SEQ. ID. NO. 8.

La présente invention a pour objet des polynucléotides ou polypeptides isolés. Un polynucléotide ou un polypeptide est dit « isolé » s'il est pris hors de son environnement original. En particulier, un polynucléotide ou un polypeptide est isolé s'il est séparé du matériel biologique avec lequel il coexiste dans le système naturel.

Selon l'invention, les séquences polynucléotidiques qui codent pour les polypeptides ou protéines de l'invention, et des fragments ou des protéines de fusion de ces polypeptides ou protéines, peuvent être utilisées pour générer des molécules d'ADN recombinant qui dirigent l'expression de ces polypeptides ou protéines, ou d'une portion active de ceux-ci, dans des cellules hôtes appropriées. Alternativement, des séquences polynucléotidiques qui s'hybrident avec des portions des séquences de polynucléotides selon l'invention peuvent également être utilisées dans des essais d'hybridation d'acides nucléiques, Southern blot, Northern blot, etc.

A cause de la dégénérescence du code génétique, d'autres séquences d'ADN codant substantiellement pour la séquence en acides aminés des polypeptides ou protéines de l'invention peuvent être utilisées pour le clonage et l'expression desdits polypeptides ou protéines. De telles séquences d'ADN incluent celles capables d'hybrider les séquences polynucléotidiques des polynucléotides de l'invention dans certaines conditions de stringence qui peuvent être ajustées de plusieurs manières. Par exemple, lors de réaction de polymérisation en chaîne (PCR), la température à laquelle s'hybrident les amorces à la matrice ou les concentrations de $MgCl_2$ dans le tampon réactionnel, peuvent être ajustés. Lors de l'utilisation de fragments d'ADN radio-marqués, ou d'oligonucléotides pour sonder des membranes, la stringence peut être ajustée en changeant les forces ioniques des solutions de lavage ou en contrôlant avec précaution la température de lavage.

L'invention comprend en particulier les polynucléotides présentant une homologie d'au moins 75 % avec le polynucléotide de séquence SEQ. ID. NO. 4, SEQ. ID. NO. 5 ou SEQ. ID. NO. 9. Le degré d'homologie exprimé en % est calculé comme suit :

$$100 - 100 \text{ x } (N'/N)$$

avec N' représentant le nombre de nucléotides modifiés par rapport à la séquence SEQ. ID. NO. 4, SEQ. ID. NO. 5 ou SEQ. ID. NO. 9 et N le nombre de nucléotides de SEQ. ID. NO. 4, SEQ. ID. NO. 5 ou SEQ. ID. NO. 9.

De manière préférentielle, une telle séquence nucléotidique homologue s'hybride spécifiquement avec la séquence complémentaire à la séquence SEQ. ID. NO. 4, SEQ. ID. NO. 5 ou SEQ. ID. NO. 9 dans des conditions stringentes. Les paramètres définissant les conditions de stringence dépendent de la température à laquelle 50 % des brins appariés se séparent ($T_m$).

Pour les séquences comprenant plus de 30 bases, et d'après Sambrook et coll. (*Molecular cloning : A Laboratory Manual,* Cold Spring Harbor Laboratories, Cold Spring Harbor, NY, **1989**), $T_m$ est définie par la relation :

$$T_m = 81{,}5 + 0{,}41 \text{ x } (\%\ G{+}C) + 16{,}6 \text{ x } \log[\text{cations}] - 0{,}63 \text{ x } (\%\ \text{formamide}) - (600 / \text{nombre de bases})$$

Pour la présente invention, les conditions de stringence seront dites « fortes » lorsque l'on utilise une température d'hybridation de 10° C en dessous de $T_m$ et des tampons d'hybridation contenant une solution 6 x SSC (chlorure de sodium 0,9 M et citrate de sodium 0,09 M). Dans de telles conditions, les polynucléotides de séquences aspécifiques ne s'hybrideront pas avec le polynucléotide de la séquence complémentaire à la séquence SEQ. ID. NO. 4, SEQ. ID. NO. 5 ou SEQ. ID. NO. 9.

Des séquences d'ADN altérées qui peuvent être utilisées en accord avec la présente invention incluent des délétions, des additions ou des substitutions de différents résidus nucléotidiques résultant dans une séquence qui code le même produit du gène ou de fonction équivalente. Le produit du gène peut également contenir des délétions, des additions ou des substitutions de résidus d'acides aminés dans les séquences des protéines de l'invention, qui résultent dans des changements dits silencieux, produisant ainsi des polypeptides et protéines de fonction équivalente.

De telles substitutions en acides aminés peuvent être réalisés sur la base de la polarité, de la charge, de la solubilité, de l'hydrophobicité, de l'hydrophilicité, et/ou de la nature amphipatique des résidus impliqués.

Par exemple, des acides aminés chargés négativement incluent l'acide aspartique et l'acide glutamique, des acides aminés chargés positivement incluent la lysine et l'arginine, des acides aminés avec des groupements polaires ayant des valeurs d'hydrophobicité voisines incluent la leucine, l'isoleucine, la valine ; la glycine, l'alanine ; l'asparagine, la glutamine ; la serine, la thréonine ; la phénylalanine, la tyrosine.

Les séquences d'ADN de la présente invention peuvent être modifiées pour altérer les séquences des polynucléotides selon l'invention pour de nombreuses raisons incluant de manière non limitative des altérations qui modifient le processus et l'expression du produit du gène. Par exemple, des mutations peuvent être introduites en utilisant des techniques bien connues de l'homme du métier, par exemple la mutagénèse dirigée, l'insertion de nouveaux sites de restriction, l'altération des glycosylations, la phosphorylation, etc.

En particulier, dans certains systèmes d'expression comme la levure, la cellule hôte peut sur-glycosyler le produit du gène. Dans un tel système, il est préférable d'altérer les séquences polynucléotidiques pour éliminer les sites de glycosylation. Dans l'étendue de la divulgation de la présente invention figurent également des séquences polynucléotidiques modifiées liées à des séquences hétérologues pour coder une protéine de fusion. La protéine de fusion peut être modifiée pour contenir un site de clivage localisé entre la séquence de la protéine selon l'invention (par exemple la séquence SEQ. ID. NO. 8) et la séquence de la protéine hétérologue, de telle sorte que la séquence de la protéine selon l'invention puisse être clivée de la partie hétérologue.

<u>Polynucléotides associés à la modulation de la prolifération cellulaire :</u>

Tout polynucléotide qui code pour un polypeptide associé à la modulation de la prolifération cellulaire ou une portion ou un variant de celui-ci comme décrit ici est couvert par la présente invention. De tels polynucléotides peuvent être simple brin (codant ou anti-sens) ou double brin et peuvent être de l'ADN (génomique, ADNc ou synthétique) ou des molécules d'ARN.

Les polynucléotides associés à la différenciation peuvent être préparés, en utilisant n'importe quelle technique disponible pour l'homme du métier. Par exemple, un tel polynucléotide peut être amplifié *via* une réaction de polymérisation en chaîne (PCR) à partir d'ADNc préparé à partir de cellules ou de tissus humains. Pour cette approche, des amorces spécifiques peuvent être dessinées et commandées ou synthétisées ; ces amorces sont basées sur la séquence dudit polynucléotide. Une portion amplifiée peut ensuite être utilisée pour isoler le gène complet à partir d'une banque humaine d'ADN génomique ou à partir d'une banque d'ADNc de n'importe quelle cellule ou n'importe quel tissu, qu'il soit normal, tumoral ou différencié, grâce à des techniques bien connues de l'homme du métier et brièvement rappelées ci-dessous. Alternativement, un gène complet peut être construit à partir de plusieurs fragments de PCR. Les molécules d'ADNc codant pour une protéine associée à la différenciation, ou une portion de celle-ci, peuvent également être préparées en criblant une banque d'ADNc obtenue par exemple à partir d'ARNm de cellules ou de tissus. De telles librairies peuvent être disponibles dans le commerce ou peuvent être préparées en utilisant les techniques classiques (cf. Sambrook et coll., *Molecular cloning: A Laboratory Manual,* Cold Spring Harbor Laboratories, Cold Spring Harbor, NY, **1989**).

Alternativement, d'autres techniques de criblage bien connues par l'homme du métier peuvent être employées.

Une molécule d'ADNc associée à la différenciation peut être séquencée en utilisant les techniques classiques utilisant des enzymes comme le fragment de Klenow de l'ADN polymérase I, la Séquenase X (US Biochemical Corp., Cleveland, OH, Etats-Unis), la Taq polymérase (Perkin Elmer, Foster City, CA, Etats-Unis), la polymérase thermostable T7 (Amersham, Chicago, IL, Etats-Unis) ou une combinaison de polymérases recombinantes et d'exonucléases à activité de relecture comme le système d'amplification Elongase (Gibco BRL, Gaithersburg, MD, Etats-Unis). Un système de séquençage automatique peut être utilisé à l'aide des instruments disponibles chez des fournisseurs commerciaux comme Perkin Elmer et Pharmacia.

La séquence partielle d'un ADNc peut être utilisée pour identifier une séquence polynucléotidique qui code pour la protéine complète associée à la modulation de la prolifération cellulaire en utilisant des techniques classiques bien connues de l'homme du métier. Parmi ces techniques, une librairie d'ADNc est criblée en utilisant une ou plusieurs sondes polynucléotidiques en utilisant les propriétés de recombinaison de RecA (ClonCapture cDNA Selection Kit, Clontech Laboratories, Etats-Unis).

Pour les techniques d'hybridation, une séquence partielle peut être radiomarquée (par exemple par translation de coupure ou par marquage des extrémités en utilisant du $^{32}P$ ou du $^{33}P$) en utilisant des techniques classiques. Une librairie de bactéries ou de bactériophages est ensuite criblée par hybridation sur des filtres contenant les colonies bactériennes dénaturées (ou les empreintes contenant les plaques de phages) avec la sonde marquée (cf. Sambrook et coll., *Molecular cloning: A Laboratory Manual,* Cold Spring Harbor Laboratories, Cold Spring Harbor, NY, **1989**). Les colonies positives ou plaques sont ensuite sélectionnées et amplifiées et l'ADN est isolé pour des analyses futures.

La séquence complète peut ensuite être déterminée en utilisant des techniques standard. Les séquences chevauchantes sont ensuite assemblées en une séquence unique continue. Une molécule d'ADNc complète peut être générée par ligature des fragments d'intérêt en utilisant des techniques classiques.

Alternativement, il existe de nombreuses techniques basées sur l'amplification pour l'obtention d'une séquence codante complète à partir d'une séquence partielle d'ADNc. Parmi elles, l'amplification est généralement réalisée via PCR. L'ensemble des kits disponibles dans le commerce peut être utilisé pour les étapes d'amplification. Les amorces peuvent être dessinées en utilisant, par exemple, des logiciels bien connus dans le métier. Les amorces nucléotidiques sont de préférence des molécules de 20 à 30 nucléotides ayant un contenu en guanine et cytosine d'au moins 50 % et qui s'hybrident avec la séquence cible à des températures comprises entre 50 et 72° C. La région amplifiée peut être séquencée comme décrit ci-dessus et les séquences chevauchantes assemblées en une séquence continue.

Parmi les approches alternatives, des séquences adjacentes à la séquence partielle peuvent être retrouvées par amplification avec une amorce de la séquence de liaison et une amorce spécifique d'une région connue. Les séquences amplifiées sont ensuite soumises à un second cycle d'amplification.

Des techniques additionnelles incluent la PCR de capture (Lagerstrom et coll., *PCR Methods Applic.* (1991), **1,** 111-19) et la PCR progressive (Parker et coll., *Nucl. Acids. Res.* (1991), **19,** 3055-60). D'autres méthodes utilisant l'amplification peuvent également être employées pour l'obtention d'une séquence complète d'ADNc.

Il est possible d'obtenir une séquence d'ADNc complète en analysant les séquences déposées dans les bases publiques « Expressed Sequence Tags » (ESTs) disponibles à partir de GenBank. Des recherches de recouvrement des ESTs peuvent être réalisées en utilisant des programmes informatiques bien connus de l'homme du métier (par exemple NCBI BLAST) et de tels ESTs peuvent être utilisés pour générer une séquence complète continue.

Les variants des séquences polynucléotidiques décrites plus haut (notamment des séquences SEQ. ID. NO. 4, SEQ. ID. NO. 5 et SEQ. ID. NO. 9) sont également inclus dans le champ de la présente invention. Les variants polynucléotidiques peuvent contenir une ou plusieurs substitutions, délétions ou insertions (cf. aussi supra dans la partie intitulée « Polynucléotides, polypeptides et protéines selon l'invention »).

Une portion de la séquence complémentaire de la séquence codante (i.e. un polynucléotide anti-sens) peut également être utilisée comme sonde ou comme modulateur de l'expression génique. Les construits d'ADNc pouvant être transcrits en ARN anti-sens peuvent être introduits dans des cellules ou dans des tissus pour faciliter la production d'ARN anti-sens. Un polynucléotide anti-sens peut être utilisé, comme décrit ici, pour inhiber l'expression d'un gène associé à la modulation de la prolifération cellulaire. La technologie anti-sens peut être utilisée pour contrôler l'expression génique en formant une triple-hélice, qui compromet la capacité de la double hélice à s'ouvrir suffisamment pour la fixation des polymérases, des facteurs de transcription ou des molécules de régulation (cf. Gee et coll. dans Huber et Carr, *Molecular and Immunologic Approaches* (1994), Futura Publishing Co., Mt. Kisco, NY). Alternativement, une molécule anti-sens peut être utilisée pour s'hybrider avec une région de contrôle du gène (par exemple un promoteur ou un site d'initiation de la transcription) et bloquer la transcription du gène, ou bloquer la traduction en inhibant la fixation des ribosomes au transcrit.

Les polynucléotides peuvent ensuite être modifiés pour augmenter leur stabilité *in vivo.* Des modifications possibles incluent (mais ne sont pas limitées à) : l'addition de séquences aux extrémités 5' et/ou 3' ; l'utilisation de phosphorothioate ou de 2' O-méthyle plutôt que des liaisons phosphodiestérase dans le squelette; et/ou l'introduction de bases comme l'inosine, la quéosine et la wybutosine de même que l'acétyladénine, la méthylthioadénine et d'autres formes modifiées de l'adénine, la cytidine, la guanine, la thymine et l'uridine.

D'autres variations des polynucléotides de la présente invention ont par ailleurs déjà été décrites précédemment dans la partie intitulée « Polynucléotides, polypeptides et protéines selon l'invention ».

Les séquences de nucléotides comme décrites dans la présente invention peuvent être jointes à d'autres séquences nucléotidiques en utilisant des techniques établies d'ADN recombinant. Par exemple, un polynucléotide peut être cloné dans un large panel de vecteurs d'expression, incluant des plasmides, des phagemides, des dérivés du phage lambda et des cosmides. Les vecteurs d'intérêt particulier incluent les vecteurs d'expression, des vecteurs de réplication et des vecteurs de séquençage. En général, un vecteur contient une origine de réplication fonctionnelle dans au moins un organisme, des sites de restriction endonucléasiques convenables et un ou plusieurs marqueurs de sélection. La présence d'autres éléments dépendra de l'utilisation spécifique souhaitée par l'homme du métier qui sélectionnera les caractéristiques du vecteur d'expression en fonction de ses besoins et des techniques disponibles.

Les polynucléotides peuvent être formulés pour entrer dans la cellule et exprimer le polypeptide correspondant. De telles formulations sont particulièrement utiles en thérapeutique comme décrit ci-après.

Les hommes du métier apprécieront qu'il existe plusieurs moyens pour exprimer un polynucléotide dans une cellule cible, et que n'importe quelle technique adéquate peut être employée. Par exemple, un polynucléotide peut être incorporé dans un vecteur viral comme un adénovirus ou un rétrovirus (mais aussi dans d'autres). Des techniques pour incorporer de l'ADN dans de tels vecteurs sont bien connues de l'homme de l'art. Un vecteur rétroviral peut transférer ou incorporer un gène pour un marqueur de sélection et/ou une entité de ciblage comme un gène codant pour le ligand d'un récepteur spécifique d'une cellule cible, afin de rendre le vecteur cible-spécifique.

D'autres formulations pour les polynucléotides incluent les systèmes de dispersion colloïdaux comme des complexes macromoléculaires, nano-capsules, microsphères, billes, et des systèmes basés sur l'utilisation des lipides incluant les émulsions huile/eau, micelles, micelles mixtes et liposomes. Le système colloïdal préféré pour une utilisation de délivrance du produit *in vitro* et *in vivo* est le liposome (i.e. une vésicule membranaire artificielle).

Polypeptides associés à la modulation de la prolifération cellulaire :

Dans l'étendue de la divulgation, les polypeptides de la présente invention comprennent au moins une portion de la protéine associée à la modulation de la prolifération cellulaire ou d'un variant de celle-ci, ladite portion étant immunologiquement et/ou biologiquement active. De tels polypeptides peuvent avoir n'importe quelle longueur, incluant la protéine complète, un oligopeptide (i.e. consistant en un nombre relativement limité d'acides aminés, comme 8-10 résidus, joints par des liaisons peptidiques) ou un peptide de taille intermédiaire. Un polypeptide peut également comprendre des séquences additionnelles.

Un polypeptide est immunologiquement actif, dans le contexte de la présente invention, s'il est reconnu par un récepteur de surface des cellules B et/ou T. L'activité immunologique peut être testée en utilisant des techniques classiques comme celles résumées par Paul, *Fundamental Immunology,* 3rd ed., 243-247 (Raven Press, 1993). De telles techniques incluent le criblage des polypeptides dérivés du polypeptide natif pour déterminer sa capacité à réagir avec un antisérum antigène-spécifique et/ou des lignées de cellules T ou des clones pouvant être préparés selon des méthodes traditionnelles. Une portion immunologiquement active de la protéine associée à la modulation de la prolifération cellulaire réagit avec de tels antisérums et/ou des cellules T et n'est pas significativement plus faible que la réactivité du polypeptide complet (e. g. dans un essai ELISA et/ou dans un essai de réactivité des cellules T). De tels criblages peuvent généralement être réalisés en utilisant des méthodes bien connues de l'homme du métier comme celles décrites dans Harlow et Lane, *Antibodies: A Laboratory Manual,* Cold Spring Harbor Laboratory, 1988.

Les épitopes des cellules B et T peuvent également être prédits en utilisant des techniques informatiques.

Alternativement, des portions immunogéniques peuvent être identifiées en utilisant une analyse informatique comme le programme Tsites® (cf. Rothbard et Taylor, *EMBO J.* (1988), **7**, 93-100 ; Deavin et coll., *Mol. Immunol.* (1996), **33**, 145-155), qui recherche des motifs peptidiques ayant le potentiel d'éliciter une réponse. Des motifs peptidiques appropriés pour la fixation aux complexes majeurs d'histo-compatibilité de classe I ou II (MHC) murins ou humains peuvent être identifiés selon la méthode BIMAS (Parker et coll., J. Immunol. (1994), 152 :163, 1994). Pour confirmer l'immunogénicité, un peptide peut être testé en utilisant une souris transgénique HLA A2 et/ou un essai *in vitro* de stimulation en utilisant des cellules dendritiques, des fibroblastes ou des cellules du sang périphérique.

De manière similaire, un polypeptide est « biologiquement actif » s'il possède une ou plusieurs fonctions structurales, régulatrices et/ou biochimiques à partir de la protéine native associée à la modulation de la prolifération cellulaire. La présence d'une activité biologique peut être déterminée selon des méthodes bien connues de l'homme du métier.

Par exemple, des études de comparaison de séquences peuvent indiquer une activité biologique particulière de la protéine. Les essais tendant à évaluer ladite activité peuvent alors être mis en oeuvre sur la base d'essais déjà connus dans le métier.

Certaines portions et d'autres variants de telles protéines devraient également montrer cette propriété selon un test *in vitro* ou *in vivo.*

Comme déjà mentionné, les polypeptides selon la présente invention peuvent comprendre une ou plusieurs portions d'un variant de la protéine endogène où la portion est immunologiquement et/ou biologiquement active (i.e. la portion présente une ou plusieurs caractéristiques antigéniques, immunogéniques et/ou biologique de la protéine complète). De préférence, une telle portion est au moins aussi active que la protéine totale lors d'essais permettant la détection de telles propriétés. Un polypeptide « variant » est un polypeptide qui diffère de la protéine native par des substitutions, des insertions, des délétions et/ou des modifications en acides aminés. Certains variants contiennent des substitutions conservatrices. « Une substitution conservatrice » est une substitution dans laquelle un acide aminé est substitué par un autre acide aminé ayant les mêmes propriétés, comme celles déterminées par l'homme du métier qui n'attend aucun changement dans la structure secondaire, ainsi que dans la nature hydropathique du polypeptide. Les substitutions d'acide aminé peuvent généralement être réalisées sur la base de similarité de polarité, de charge, de solubilité, d'hydrophobicité, d'hydrophylicité, et/ou de la nature amphipathique des résidus. Par exemple, des acides aminés chargés négativement incluent l'acide aspartique et l'acide glutamique ; des acides aminés chargés positivement incluent la lysine et l'arginine ; et des acides aminés non chargés polaires ayant des valeurs d'hydrophobicité similaire incluent la leucine, l'isoleucine et la valine ; la glycine et l'alanine ; l'asparagine et la glutamine ; la sérine, la thréonine, la phénylalanine et la tyrosine. D'autres groupes d'acides aminés qui peuvent représenter des changements conservateurs sont notamment les suivants : (1) Ala, Pro, Gly, Glu, Asp, Gln, Asn, Ser, Thr ; (2) Cys, Ser, Tyr, Thr (3) Val, Ile, Leu, Met, Ala, Phe ; (4) Lys, Arg, His ; et (5) Phe, Tyr, Trp, His. Un variant peut également, ou alternativement, contenir des changements non conservateurs.

Des variants faisant partie de cette invention incluent également des polypeptides dans lesquels la structure primaire de la protéine native est modifiée par formation de conjugués covalents ou pas avec d'autres polypeptides ou des structures chimiques comme des groupes lipidiques ou des groupes glycosyle, ou phosphate acétyle.

La présente invention inclut également des polypeptides avec ou sans motifs de glycosylation. Les polypeptides exprimés dans des systèmes d'expression de levure ou de cellules de mammifères peuvent être, en termes de poids moléculaire et de schéma de glycosylation, similaires à ou légèrement différents de la molécule native selon le système d'expression utilisé.

L'expression d'ADN chez la bactérie comme *E. Coli* conduit à des molécules non-glycosylées. Les sites de N-glycosylation des protéines eucaryotes sont caractérisés par le triplet d'acides aminés Asn-A1-Z où A1 est n'importe quel acide aminé excepté Pro, et Z est une sérine ou une thréonine.

Également inclus dans la présente invention sont les allèles de la protéine associée à la modulation de la prolifération cellulaire. Les allèles sont des formes alternatives de la protéine native résultant d'une ou plusieurs mutations (pouvant être des acides aminés supprimés, ajoutés ou substitués), conduisant à un ARN altéré. Les protéines allèliques peuvent différer dans la séquence, mais la structure globale ainsi que la fonction sont substantiellement similaires. La protéine associée à la modulation de la prolifération cellulaire, les variants et les portions de celle-ci peuvent généralement être préparés à partir d'acides nucléiques codant le polypeptide souhaité en utilisant des techniques classiques. Pour préparer la protéine endogène, un ADNc isolé peut être utilisé.

D'autres variations des polypeptides et protéines de la présente invention ont par ailleurs déjà été décrites précédemment dans la partie intitulée « Polypeptides et polynucléotides selon l'invention ».

Pour préparer un variant polypeptidique, des techniques standard de mutagenèse, comme la mutagenèse dirigée en utilisant un oligonucléotide dirigé, peuvent être utilisées.

En général, n'importe quel vecteurs d'expression connus des hommes de l'art peuvent être employés pour exprimer des polypeptides recombinants de cette invention. L'expression peut être obtenue dans n'importe quelle cellule hôte appropriée qui a été transformée ou transfectée avec un vecteur d'expression contenant une séquence d'ADN qui code pour le polypeptide recombinant. Des cellules hôtes convenables incluent des cellules procaryotes, d'eucaryotes supérieurs ou de levure. De préférence, les cellules hôtes employées sont *E. Coli,* des cellules de levure ou des cellules de mammifères comme COS, CHO, MCF7 (cellules tumorales humaines isolées à partir d'un carcinome mammaire) ou DU 145 (cellules tumorales humaines isolées à partir d'un cancer de la prostate).

Après expression, le surnageant des systèmes hôtes/vecteur qui sécrètent la protéine recombinante ou le polypeptide dans le milieu de culture peut être dans un premier temps concentré en utilisant des techniques classiques comme la précipitation alcoolique ou la filtration à l'aide de filtres du commerce. Après l'étape de concentration, le produit résultant peut être appliqué sur une matrice de purification adéquate comme une matrice d'affinité ou une résine échangeuse d'ions. Une ou plusieurs étapes de HPLC peuvent être employées pour poursuivre la purification du polypeptide.

Certaines portions et d'autres variants peuvent également être générés par des moyens synthétiques en utilisant des techniques bien connues de l'homme de l'art. Par exemple, des portions et autres variants ayant moins de 500 acides aminés, de préférence moins de 100 acides aminés et plus préférentiellement moins de 50 acides aminés peuvent être synthétisés par voie chimique. Les polypeptides peuvent être synthétisés en utilisant des techniques de synthèse sur phase solide disponibles commercialement, comme la méthode de synthèse sur résine de Merrifield où les acides aminés sont séquentiellement ajoutés à une chaîne d'acides aminés en cours de synthèse (cf. Merrifield, *J. Am. Chem. Soc.* (1963), **85**, 2149-2146). De nombreuses autres techniques de synthèse sur phase solide sont également disponibles (par exemple la méthode de Roberge et coll., *Science* (1995), **269,** 202-204). Des équipements pour la synthèse automatique de polypeptides sont commercialement disponibles chez des fournisseurs comme Applied Biosystems, Inc. (Foster City, CA, Etats-Unis) ; la synthèse des polypeptides peut alors être réalisée en suivant les recommandations du constructeur.

Polynucléotides ou polypeptides isolés:

En général, les polypeptides et polynucléotides décrits dans la présente invention sont isolés. Un polypeptide ou un polynucléotide « isolé » est un polynucléotide ou un peptide enlevé de son environnement original. Par exemple, une protéine naturelle est isolée si elle est séparée du matériel biologique avec lequel elle coexiste dans le système naturel. Un polynucléotide est considéré comme isolé si, par exemple, il est cloné dans un vecteur qui ne fait pas partie de l'environnement naturel.

Séquences signal :

Les méthodes pour prédire si une protéine possède une séquence signal, de même que pour prédire le point de clivage pour cette séquence, sont disponibles. Par exemple, la méthode de McGeoch *(Virus Res.* (1985), **3,** 271-286) utilise l'information d'une courte séquence chargée N-terminale d'une région non chargée de la protéine (non clivée) complète. La méthode de Von Heinje (*Nucleic Acid Res.* (1986), **14**, 4683-4690) utilise l'information des résidus qui entourent le site de clivage. La précision de la prédiction des points de clivage des protéines sécrétées connues de mammifères pour chacune de ces méthodes est de 75-80 %. Cependant, les deux méthodes ne prédisent pas toujours les mêmes sites de clivage pour une protéine donnée.

Dans le cas présent, la séquence déduite d'acides aminés du polypeptide sécrété a été analysée par un programme informatique appelé SignalP (Sequence Analysis Software Package of the Genetic Computer Group, University of Wisconsin Biotechnology Center, 1710 University Avenue, Madison, WI 53705, Etats-Unis). Ce programme informatique prédit la localisation cellulaire d'une protéine basée sur la séquence en acides aminés.

Il est également reconnu que, dans certains cas, le clivage de séquence signal d'une protéine sécrétée n'est pas uniforme, résultant ainsi en la formation de plusieurs espèces moléculaires. Ces polypeptides et les polynucléotides codant pour ces polypeptides sont couverts par la présente invention.

De plus, la séquence signal identifiée par l'analyse mentionnée ci-dessus peut ne pas être prédictive de la séquence signal naturelle. Par exemple, la séquence signal naturelle peut être en amont ou en aval de la séquence prédite. Cependant, il est vraisemblable que ladite séquence signal sera capable de diriger la protéine sécrétée vers le réticulum endoplasmique. Ces polypeptides et les polynucléotides codant pour ces polypeptides sont couverts par la présente invention.

Les sites de clivage varient quelques fois d'une espèce à l'autre et ne peuvent pas être prédits avec certitude.

Les séquences d'acides aminés, démarrant par la méthionine, sont identifiées par des séquences déterminées codées par des polynucléotides, bien que d'autres phases de lecture puissent être facilement traduites en utilisant des techniques de biologie moléculaire bien connues de l'homme du métier. Les polypeptides produits par ces phases ouvertes de lecture alternative sont également envisagés par cette présente invention.

<u>Anticorps et fragments de ceux-ci :</u>

La présente invention fournit des agents de fixation, comme les anticorps qui fixent spécifiquement la protéine associée à la modulation de la prolifération cellulaire. Un tel agent est dit comme « fixant spécifiquement » à la protéine de modulation de la prolifération cellulaire s'il réagit à un niveau détectable (par exemple par un essai ELISA) avec une protéine associée à la modulation de la prolifération cellulaire ou une portion ou un variant de celle-ci et ne réagit pas de manière détectable avec d'autres protéines. « La fixation » se réfère à une association non covalente entre 2 molécules séparées de telle sorte qu'un complexe se forme. La capacité à la fixation peut être évaluée, par exemple, par la détermination de la constante de fixation pour la formation du complexe. La constante de fixation est la valeur obtenue lorsque la valeur de la concentration du complexe est divisée par le produit des valeurs des concentration des composants. En général, 2 produits sont dits « fixés » lorsque la constante de fixation atteint 103 1/mol. La constante de fixation peut être déterminée en utilisant des méthodes bien connues de l'homme du métier.

N'importe quel agent capable de répondre aux critères ci-dessus peut être considéré comme un agent fixant.

Dans la présente invention, un agent de fixation est de préférence un anticorps ou un fragment de celui-ci. Les anticorps peuvent être préparés par n'importe quelle technique disponible à l'homme du métier (cf. Harlow et Lane, *Antibodies. A laboratory Manual,* Cold Spring Harbor Laboratory, 1988). En général, les anticorps peuvent être produits par des techniques de culture cellulaire incluant la génération d'anticorps monoclonaux ou *via* des transfections de gènes d'anticorps dans des cellules hôtes de bactéries ou de mammifères afin de produire des anticorps recombinants.

Parmi d'autres techniques, on préférera employer celles décrites ci-après. Un immunogène contenant le polypeptide est injecté chez un groupe de mammifères (par exemple souris, rats, lapins, moutons ou chèvres). Dans cette étape, les polypeptides de la présente invention peuvent servir d'immunogènes sans modification. Alternativement, et particulièrement pour des peptides de petite taille, une réponse immunitaire supérieure peut être induite si le polypeptide est joint à une protéine de transport comme l'albumine de sérum bovin ou l'hémocyanine de patelle. L'immunogène est injecté chez l'animal hôte, de préférence selon un schéma prédéterminé, et les animaux sont saignés périodiquement. Des anticorps polyclonaux spécifiques du polypeptide peuvent ainsi être purifiés à partir de tels antisérums, par exemple par chromatographie d'affinité en utilisant le peptide couplé à un support solide adéquat.

<u>Protéines de fusion :</u>

N'importe quel gène de fusion peut être réalisé par l'homme du métier pour analyser la localisation sub-cellulaire d'une protéine selon l'invention, en particulier la localisation sub-cellulaire de la protéine de séquence SEQ. ID. NO. 8. De nombreuses constructions plasmidiques sont disponibles commercialement comme la protéine Glutathione S Transférase (GST) ou des protéines fluorescentes comme la Green Fluorescent Protein (GFP) ou encore et de manière non exhaustive un marquage poly-Histidine.

Des cellules hôtes eucaryotes humaines (par exemple HEK-293) sont sous-cultivées durant 24 h avant le protocole de transfection permettant un métabolisme normal des cellules et une meilleure efficacité de transfection. Des concentrations croissantes (1, 5 et 10 μg) de vecteur seul contenant la protéine révélatrice (GFP, GST ou Tag Histidine) ou de vecteur contenant le polynucléotide de séquence SEQ. ID. NO. 4, le polynucléotide de séquence SEQ. ID. NO. 5 ou le polynucléotide de séquence SEQ. ID. NO. 9 fusionnée avec la protéine révélatrice ont été réalisées en utilisant le réactif Effectene® selon les recommandations du fabricant (Qiagen).

Les cellules sont ensuite analysées par microscopie confocale, par exemple, pour détecter la localisation de la protéine. Si la protéine est suspectée d'être sécrétée par exemple, les surnageants sont récupérés, lyophilisés, déposé sur gel d'acrylamide et analysés par la technique de Western blot à l'aide d'anticorps dirigés contre la protéine révélatrice.

Compositions pharmaceutiques :

Selon certains aspects de l'invention, des produits tels que des polypeptides, anticorps et/ou acides nucléiques peuvent être incorporés dans des compositions pharmaceutiques ou des vaccins. Les compositions pharmaceutiques comprennent un ou plusieurs de ces produits et un ou des excipients (transporteurs) pharmaceutiquement acceptables. Certaines compositions pharmaceutiques éventuellement utilisables comme vaccins pourront comprendre un ou plusieurs polypeptides et un activateur de la réponse immunitaire, comme un adjuvant ou un liposome (dans lequel le produit est incorporé). Les compositions pharmaceutiques et les vaccins peuvent de plus contenir un système d'administration, comme des microsphères biodégradables. Les compositions pharmaceutiques et les vaccins dans l'étendue de la divulgation de la présente invention peuvent également contenir d'autres produits pouvant être biologiquement actifs ou inactifs.

Une composition pharmaceutique ou un vaccin peut contenir de l'ADN codant pour un ou plusieurs polypeptides comme décrit ci-dessus, de telle sorte que le polypeptide est généré *in situ.* Comme mentionné précédemment, l'ADN peut être présent sous n'importe quelle forme d'administration connue de l'homme du métier, y compris des systèmes d'expression d'acides nucléiques, bactériens ou viraux. Les systèmes d'expression d'acides nucléiques appropriés contiennent les séquences d'ADN nécessaires pour l'expression chez le patient.

Les systèmes d'administration basés sur une bactérie impliquent l'administration de *bacterium* (comme Bacillus-Calmette-Guerrin) qui exprime une portion immunogène du polypeptide à sa surface. De préférence, l'ADN peut être introduit en utilisant un système d'expression viral (par exemple un pox virus, un rétrovirus ou un adénovirus) impliquant l'utilisation d'agents non pathogènes (défectif).

Bien que tout transporteur adéquat connu de l'homme du métier puisse être employé dans des compositions pharmaceutiques de cette invention, le type de transporteur variera selon le mode d'administration choisi. Les compositions de la présente invention pourront être formulées pour chaque mode d'administration approprié, y compris, par exemple, les voies topique, nasale, intraveineuse, intra-craniale, intra-péritonéale, sous-cutanée et intramusculaire.

Pour une administration parentérale, comme une injection sous-cutanée, le transporteur contient de préférence de l'eau, du sel, de l'alcool, de la graisse, de la paraffine ou un tampon. Pour une administration orale, tout transporteur cité ci-dessus ou un transporteur solide, comme du mannitol, du lactose, de l'amidon, du stéarate de magnésium, du talc, de la cellulose, du glucose, du sucrose et du carbonate de magnésium peut être employé. Des microsphères biodégradables peuvent aussi être utilisées comme transporteurs pour les compositions pharmaceutiques de cette invention. Pour certaines applications topiques, des formulations comme des crèmes ou des lotions sont préférées.

De telles compositions peuvent également comprendre des tampons (par exemple des solutions salines tamponnées neutres ou phosphate), des carbohydrates (par exemple du glucose, du mannose, du sucrose ou des dextranes), du mannitol, des protéines, des polypeptides ou des acides aminés comme la glycine, des antioxydants, des agents chélateurs comme l'EDTA ou la glutathione, des adjuvants (par exemple de l'hydroxyde d'aluminium) et/ou des agents protecteurs. Alternativement, les compositions de la présente invention peuvent se présenter sous forme d'un lyophilisat. Des produits peuvent également être encapsulés dans des liposomes en utilisant des technologies classiques.

Selon l'invention, chacune des variétés d'adjuvants peut être utilisée dans des vaccins pour induire la réponse immunitaire. La plupart des adjuvants contient une substance protégeant l'antigène d'un catabolisme rapide, comme l'hydroxyde d'aluminium ou l'huile minérale et un stimulateur des réponses immunitaires comme le lipide A, des protéines dérivées de Bordetella Pertussis ou Mycobacteium tuberculosis. Des adjuvants adéquats sont commercialement disponibles comme, par exemple : l'adjuvant de Freund et l'adjuvant complet (Difco Laboratories, Detroit, MIY, Etats-Unis ; Merck Adjuvant 65 (Merck and Company, Inc., Rahway, NJ, Etats-Unis)), des microsphères biodégradables ; le lipide A monophosphoryle ; et des cytokines comme le GM-CSF ou l'interleukine-2, -7 ou -12.

Les compositions décrites ci-dessus peuvent également être administrées sous forme de formulations retard (i.e. une formulation comme une capsule ou une éponge qui déclenche la libération lente du produit après administration). De telles formulations peuvent généralement être préparées en utilisant des technologies bien connues de l'homme du métier et administrées, par exemple, par voie orale, rectale ou en implantation sous-cutanée ou par implantation sur le site cible désiré. Les formulations retard peuvent contenir un polypeptide, un polynucléotide ou un anticorps dispersé dans une matrice transporteuse et/ou contenu dans un réservoir protégé par une membrane de diffusion. Les transporteurs pour l'utilisation de telles formulations sont biocompatibles et doivent également être biodégradables ; de préférence la formulation fournit un niveau relativement constant de la libération du composant actif. La quantité de produit actif contenue dans la formulation retard dépend du site d'implantation.

Thérapie anticancéreuse :

Selon d'autres aspects de la présente invention, les produits décrits peuvent être utilisés en thérapie anticancéreuse. En particulier, les polynucléotides et polypeptides associés à la modulation de la prolifération cellulaire peuvent être utilisés pour inhiber la croissance et induire une modulation de la prolifération cellulaire dans des tumeurs spécifiques du sein ou de la prostate.

De tels polypeptides ou polynucléotides peuvent également être utilisés pour la thérapie de nombreux carcinomes incluant les mélanomes, les formes multiples de glioblastomes, les carcinomes du poumon ainsi que les cancers colorectaux. Des agents qui activent l'expression de tels polypeptides ou polynucléotides peuvent également être employés dans le cadre de ces thérapies.

Selon ces aspects de l'invention, les produits (pouvant être des polypeptides ou des acides nucléiques) sont de préférence incorporés dans des compositions pharmaceutiques comme décrit ci-dessus.

Les patients adéquats pour la thérapie sont tous les animaux à sang chaud, et de préférence l'être humain. Un patient éligible pour une thérapie selon l'invention peut ou non être diagnostiqué comme étant affecté par un cancer. Autrement dit, les compositions pharmaceutiques décrites ci-dessus peuvent ainsi être utilisées pour inhiber le développement d'un cancer à différents stades de la maladie (pour prévenir l'apparition d'un cancer ou pour traiter un patient affecté par un cancer).

Les compositions pharmaceutiques de la présente invention seront administrées de manière appropriée pour chaque cancer spécifique à traiter.

La voie, la durée et la fréquence d'administration seront déterminées en fonction de l'état du patient, du type et de la sévérité de la maladie, et de la méthode d'administration. Les voies et fréquences d'administration peuvent varier d'un individu à l'autre. En général, les compositions pharmaceutiques et les vaccins peuvent être administrés par injection (par exemple par la voie intra-cutanée, intramusculaire, intraveineuse ou sous-cutanée), par voie intra-nasale (par exemple par inhalation) ou par voie orale. De préférence, entre 1 et 10 doses peuvent être administrées sur une période de 52 semaines. Des protocoles alternatifs peuvent être appropriés pour chaque patient individuellement.

En général, un dosage approprié et un régime de traitement contiennent le produit actif en quantité suffisante pour fournir un bénéfice thérapeutique et/ou prophylactique. Une telle réponse peut être suivie par l'établissement d'un devenir clinique amélioré (par exemple des rémissions plus fréquentes, une survie en absence de la maladie complète, partielle ou plus longue) chez les patients traités comparés aux patients non traités ou traités par des doses moindres.

Selon d'autres aspects de la présente invention, un polypeptide peut être administré à des doses variant de 100 $\mu$g à 5 mg. Les molécules d'ADN codant pour de tels polypeptides peuvent généralement être administrées en quantité suffisante pour générer des niveaux comparables de polypeptides. Des dosages appropriés peuvent généralement être déterminés en utilisant des modèles expérimentaux et/ou des essais cliniques. En général, l'utilisation de la dose minimum suffisante pour fournir une thérapie efficace est préférée. Les patients peuvent généralement être suivis en ce qui concerne l'efficacité de la thérapie en utilisant des essais adéquats pour les conditions de traitement ou de prévention qui apparaîtront familiers à l'homme du métier.

Méthodes de détection et de suivi du cancer:

Les polypeptides, polynucléotides et anticorps décrits dans la présente invention peuvent être utilisés dans de nombreuses méthodes pour la détection de cancer chez un patient. La présence de polypeptides et/ou de polynucléotides associés à la modulation de la prolifération cellulaire comme ceux décrits dans la présente invention dans des cellules d'un hôte est indicative de la différenciation et de l'arrêt de la croissance cellulaire. Ainsi, les séquences associées à la modulation de la prolifération cellulaire peuvent être utilisées comme marqueurs de distinction entre des cellules normales et des cellules malignes (et permettre au médecin qui interprètera les résultats de diagnostiquer, par exemple, des carcinomes de prostate, de sein, de poumon et de colon). Les séquences associées à la modulation de la prolifération cellulaire peuvent également être utilisées comme marqueurs de suivi du traitement.

Des méthodes comprenant l'utilisation d'anticorps peuvent permettre à l'expérimentateur de détecter la présence ou l'absence d'un polypeptide décrit dans la présente invention dans n'importe quel échantillon biologique disponible. Des échantillons biologiques disponibles incluent des biopsies de tissus tumoral et de tissus sains, d'homogénat ou d'extraits de tels tissus obtenus d'un patient. Il existe un grand nombre de types d'essais connus de l'homme du métier pour l'utilisation d'anticorps pour la détection de marqueurs polypeptidiques dans un échantillon (cf., par exemple, Harlow et Lane, Antibodies: *A Laboratory Manual,* Cold Spring Harbor Laboratory, 1988).

Par exemple, un essai peut être réalisé selon la technique de Western blot, dans laquelle une préparation de protéine est soumise à un gel d'électrophorèse, transférée sur une membrane adéquate et soumise à réaction avec un anticorps. La présence d'un anticorps sur la membrane peut ensuite être détectée en utilisant un réactif de détection adéquat, comme décrit ci-dessous.

Selon d'autres aspects de l'invention, des essais comprenant l'utilisation d'un anticorps immobilisé sur un support solide fixé au polypeptide peuvent être réalisés. Le polypeptide fixé peut être détecté en utilisant un second anticorps ou d'autre réactifs contenant un groupement de révélation. Alternativement, un essai compétitif peut être utilisé, dans lequel un polypeptide est marqué avec un groupement de révélation et permet la fixation à l'anticorps immobilisé après incubation de l'anticorps avec l'échantillon. La faculté selon laquelle des composants de l'échantillon inhibent la fixation du polypeptide marqué à l'anticorps est indicative de la réactivité de l'échantillon avec l'anticorps immobilisé, et ainsi, indicative de la concentration du polypeptide dans l'échantillon.

Le support solide peut être n'importe quel matériel connu de l'homme du métier sur lequel l'anticorps peut être attaché. Par exemple, le support solide peut être un puits de test d'une plaque de microtitration ou un filtre de nitrocellulose ou toute autre membrane adéquate. Alternativement, le support peut être une bille, du verre, de la matière plastique ou du latex comme du polystyrène ou du chlorure de polyvinyle. Le support peut également être une particule magnétique.

L'anticorps peut être immobilisé sur support solide en utilisant une variété de techniques connues de l'homme du métier et largement décrites dans la littérature scientifique. Dans le contexte de la présente invention, le terme « immobilisation » se réfère aussi bien à une association non-covalente, comme l'adsorption, qu'à une liaison covalente (pouvant être une liaison directe entre l'antigène et les groupements fonctionnels sur le support ou une liaison par l'utilisation d'agent liant). L'immobilisation par adsorption à un puits d'une plaque de microtitration ou à une membrane est préférée. Dans ce cas, l'adsorption peut être accomplie en mettant en présence l'anticorps, dans un tampon adéquat, avec le support solide pendant un temps adéquat. Le temps de contact varie selon la température, mais est typiquement compris entre 1 heure et 1 jour. En général, la mise en présence du puits d'une plaque en plastique (polystyrène ou chlorure de polyvinyle, par exemple) de microtitration avec une quantité d'anticorps de 1 pg à 10 ng, et de préférence de 100 à 200 ng, est suffisante pour immobiliser une quantité adéquate du polypeptide.

Une liaison covalente de l'anticorps à un support solide peut également être créée en faisant réagir le support avec un réactif bi-fonctionnel qui réagit avec le support et le groupement fonctionnel, comme l'hydroxyle ou un groupe aminé, sur l'anticorps. Par exemple, l'anticorps peut être lié de manière covalente à des supports recouverts de polymère approprié en utilisant la benzoquinone ou par condensation d'un groupement aldéhyde sur le support avec une amine et de l'hydrogène activé sur le partenaire selon des techniques classiques.

Dans certains cas, l'essai pour la détection d'un polypeptide dans un échantillon est le test de double anticorps « sandwich ». Cet essai peut être réalisé en mettant en présence l'anticorps immobilisé sur un support solide, communément le puits d'une plaque de microtitration, avec l'échantillon biologique, de telle sorte que le polypeptide dans l'échantillon puisse fixer l'anticorps immobilisé. Les produits non fixés sont supprimés du complexe polypeptide-anticorps et un second anticorps (contenant un groupement de révélation) capable de se fixer sur différents sites du polypeptide est ajouté. La quantité d'anticorps secondaire qui reste fixé au support solide est ensuite déterminée en utilisant une méthode appropriée spécifique du groupement de révélation.

Plus spécifiquement, lorsque l'anticorps est immobilisé sur le support défini ci-dessus, les sites de fixation de la protéine sont typiquement bloqués. N'importe quel agent bloquant connu de l'homme du métier, comme de l'albumine de sérum bovin ou du Tween 20™ (Sigma Chemical Co, St. Louis, MO, Etats-Unis) peut être utilisé. L'anticorps immobilisé est ensuite incubé avec l'échantillon et le polypeptide peut fixer l'anticorps. L'échantillon peut être dilué dans un diluant adéquat, comme la solution saline tamponnée au phosphate (PBS) avant l'incubation.

En général, le temps de contact approprié (i.e. le temps d'incubation) est la période de temps suffisante pour la détection de la présence du polypeptide au sein de l'échantillon obtenu à partir d'un individu. De préférence, le temps de contact ainsi défini est suffisant pour atteindre un niveau de fixation d'au moins 95 % de celui atteint à l'équilibre entre le peptide fixé et le peptide non-fixé. L'homme du métier aura conscience que le temps nécessaire pour atteindre l'équilibre doit être déterminé en mesurant le niveau de fixation se produisant pendant une période de temps. A température ambiante, un temps d'incubation de 30 minutes est généralement suffisant. Les produits non fixés peuvent ensuite être retirés par lavage du support solide avec un tampon approprié comme la solution saline tamponnée au phosphate contenant 0,1 % de Tween 20™. Le second anticorps, contenant un groupement révélateur, peut ensuite être ajouté sur le support solide.

Les groupements révélateurs comprennent de préférence des enzymes (comme la peroxydase), des substrats des cofacteurs, des inhibiteurs de colorants, des groupements luminescents, des groupements fluorescents et de la biotine. La conjugaison de l'anticorps au groupement révélateur peut être réalisée en utilisant des méthodes standard connues de l'homme du métier.

Le second anticorps est ensuite incubé avec le complexe immobilisé anticorps-polypeptide pendant un temps suffisant permettant la détection du polypeptide fixé.

Un temps approprié peut généralement être déterminé en mesurant le niveau de fixation qui se produit pendant une période de temps. Le second anticorps non fixé est ensuite retiré et le second anticorps fixé est détecté en utilisant un groupement révélateur. La méthode utilisée pour la détection du groupement révélateur dépend de la nature du groupement révélateur. Pour des groupements radioactifs, des méthodes de comptage à scintillation ou auto-radiographique sont généralement appropriées. Des méthodes spectroscopiques peuvent être utilisées pour détecter des colorants, des groupements fluorescents ou luminescents. La biotine peut être détectée en utilisant l'avidine, couplée à un groupement révélateur (communément un groupement radioactif ou fluorescent ou une enzyme). Les groupements révélateurs enzymatiques peuvent généralement être détectés par l'addition d'un substrat (généralement pendant une période de temps adéquate), suivie d'une analyse spectroscopique (ou autre) des produits de la réaction.

Pour déterminer la présence ou l'absence d'un cancer, le signal détecté du groupement révélateur fixé au support solide est généralement comparé au signal correspondant à la valeur établie à partir d'un tissu non tumoral. De préférence, la valeur limite est le signal moyen obtenu lorsque l'anticorps immobilisé est incubé avec des échantillons de patients sans cancer. En général, un échantillon générant un signal correspondant à 3 fois plus ou 3 fois moins la déviation standard de la valeur limite prédéterminée est considéré comme indicatif.

Pour plus de précisions, l'homme du métier se référera à la publication suivante : Sackett et coll., *Clinical Epidemiology. A basic Science for Clinical Medicine,* p. 106-107 (Little Brown and Co., 1985).

La présence ou l'absence d'un cancer chez un patient peut également être déterminée en évaluant les taux d'ARNm codant pour le polypeptide de la présente invention dans l'échantillon biologique (par exemple une biopsie) relative à une valeur limite prédéterminée.

Une telle évaluation peut être réalisée en utilisant un grand nombre de méthodes connues par l'homme de l'art comme, par exemple, l'hybridation *in situ* et l'amplification par réaction de polymérisation en chaîne (PCR). Les sondes et les amorces utilisées dans de telles méthodes peuvent être généralement générées à partir des séquences listées dans les exemples, ou à partir de séquences similaires identifiées dans d'autres individus. Les sondes peuvent être utilisées dans des techniques d'hybridation classiques, et peuvent être marquées avec un agent de détection pour faciliter la détection de la sonde. De tels réactifs comprennent (mais ne sont pas limités à) : des radionucléides, des colorants fluorescents et des enzymes capables de catalyser la formation de produits détectables.

Les amorces peuvent généralement être utilisées dans des méthodes de détection incluant la réaction de polymérisation en chaîne (PCR) comme la RT-PCR, dans laquelle la PCR est appliquée en conjonction avec la transcription inverse. Typiquement, l'ARN est extrait d'un tissu échantillon et est transcrit de manière inverse pour la production de molécules d'ADNc. L'amplification par PCR en utilisant des amorces spécifiques génère des molécules d'ADNc associées à la modulation de la prolifération cellulaire, pouvant être séparées et visualisées en utilisant, par exemple, le gel d'électrophorèse. L'amplification est typiquement réalisée sur des échantillons obtenus à partir de paires de tissus tumoral et non tumoral d'un même individu, ou encore à partir de paires de tissus tumoral et non tumoral d'individus différents. La réaction d'amplification peut être réalisée sur des dilutions successives d'ADNc couvrant 2 ordres de magnitude. Une modification d'un facteur 2 ou plus de l'expression dans des dilutions de l'échantillon tumoral comparé aux mêmes dilutions de l'échantillon non tumoral est typiquement considérée comme indicative.

Certains essais en vue d'établir un diagnostic peuvent par ailleurs être réalisés directement sur la tumeur.

Un tel essai implique le contact des cellules tumorales avec un anticorps ou un fragment de celui-ci qui fixe une protéine associée à la modulation de la prolifération cellulaire. L'anticorps fixé ou un fragment de celui-ci peut être détecté directement ou indirectement *via* un groupement révélateur. De tels anticorps peuvent également être utilisés dans des applications histologiques. Alternativement, un polynucléotide anti-sens peut être utilisé dans de telles applications.

Selon d'autres aspects de la présente invention, la progression et/ou la réponse à un traitement d'un cancer peut être suivie en réalisant n'importe lequel des essais décrits ci-dessus pendant une période de temps et évaluer le changement de niveau de la réponse (i.e. la quantité de polypeptide ou d'ARNm détectée). Par exemple, les essais peuvent être réalisés tous les mois pendant une période de 1 à 2 ans. En général, un cancer progresse chez les patients dans lesquels le niveau de la réponse diminue au cours du temps. Par opposition, un cancer ne progresse pas lorsque le signal détecté reste constant ou augmente au cours du temps.

La présente invention fournit également des kits pour l'utilisation de l'ensemble des méthodes de diagnostic décrites ci-dessus. De tels kits comprennent 2 composants (ou plus) nécessaires à la réalisation de tels essais. De tels composants peuvent être les produits, les réactifs et/ou les récipients ou équipements. Par exemple, un récipient à l'intérieur d'un kit peut contenir un anticorps monoclonal ou un fragment de celui-ci qui fixe spécifiquement un polypeptide associé à la modulation de la prolifération cellulaire. De tels anticorps ou fragments peuvent être fournis attachés sur un matériel support comme décrit ci-dessus. Un ou plusieurs récipients additionnels peuvent contenir des éléments, comme des réactifs ou des tampons, pour être utilisés dans l'essai. De tels kits peuvent également contenir un réactif de détection (par exemple un anticorps) contenant un groupement révélateur convenable pour une détection directe ou indirecte de l'anticorps fixé.

<u>Méthodes pour l'identification d'agents de fixation ou de modulation :</u>

La présente invention fournit en outre des méthodes pour l'identification de produits qui se fixent et/ou qui modulent l'expression de protéines associées à la modulation de la prolifération cellulaire. De tels agents peuvent généralement être identifiés lors de la mise en contact d'un polypeptide fourni dans la présente invention avec un produit / un agent candidat dans des conditions et pendant un temps suffisant pour permettre l'interaction avec le polypeptide et/ou son effecteur (par exemple son récepteur). Chaque variété d'essai de fixation bien comme de l'homme du métier peut ainsi être réalisée pour tester la capacité d'un produit candidat à la fixation au polypeptide ou à son effecteur (par exemple son récepteur).

Dans d'autres essais, les agents peuvent être criblés en utilisant des cellules connues comme ayant une augmentation de l'expression du gène associé à la modulation de la prolifération cellulaire. De telles cellules peuvent être mises en contact avec des agents candidats et l'expression du gène associé à la modulation de la prolifération cellulaire évaluée par rapport à l'expression observée en absence d'agent candidat.

Alternativement, des produits candidats peuvent être criblés pour leur habilité à moduler l'expression (par exemple la transcription) d'une protéine associée à la modulation de la prolifération cellulaire. Pour évaluer l'effet d'un agent candidat sur l'expression de la protéine associée à la modulation de la prolifération cellulaire, un promoteur ou un élément de régulation de celui-ci peut être lié à un gène de révélation comme décrit ci-dessus. Un tel construit peut être transfecté dans des cellules hôtes convenables, lesquelles seront alors mises en contact avec l'agent candidat.

Lesdites cellules hôtes seront de préférence utilisées pour le criblage de librairies de petites molécules issues de programmes de chimie combinatoire. Selon cette variante préférée, les cellules sont incubées avec la librairie ; les cellules sont ensuite lysées et le surnageant est analysé pour l'activité du gène de révélation selon des protocoles standard.

Les produits qui augmentent l'activité du gène de révélation sont des inducteurs de la transcription du gène associé à la modulation de la prolifération cellulaire et peuvent ainsi être utilisés pour inhiber la progression du cancer.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

## EXEMPLES :

Exemple 1 : isolement et caractérisation des fragments d'ADNc SEQ. ID. NO. 4 et SEQ. ID. NO. 5

La séquence d'ADN SEQ. ID. NO. 1, présente dans la base publique contenant les ESTs a été retenue (Genbank, numéro d'accès AA176076), et est reproduite ci-après :

```
  1 ctccatgana tgccactgtt cctgaccagc cgaagtccag gcagggggag gtttccttgt
 61 ccaggaggga gaggacctcc accctctgac ctttcagttt tccatagcag cccagtgtga
121 agctcggcag ngggtgggca gggcatcagg acaagatgaa gatgctggct ctggaatgag
181 gactcccctt ggcataaggg gtagtggctg gcagaggttg cccagctctt ttggaggcca
241 cagggaatag aggtggggtg gtgcttccgt tcctgtgagg cccgcccccc tgagcccccct
301 ccgtctgctt cttgtttggc agaaatagat gtggggcaca gcctaagaat gatgggaatc
361 tgcccaagct gtgccctctg gggaaatgaa atcttgctcc tcgtgcagct tccccttttc
421 ccttgcgggg tgggggctgg gggtgccaag gctccaccct tggaaggggt tgctggggtg
481 ccaagctcca acctttgctc gctggcagaa ggaaggaagc acctcctgta tctttcaggg
541 ncctgaagcc antttgcctc agagaagaga nagtcc
```

A partir de cette séquence, un couple d'amorces 5' Fwdl et 3' Rev1 de séquences respectives SEQ. ID. NO. 2 et SEQ. ID. NO. 3 (reproduites ci-dessous) a été synthétisé afin d'obtenir une sonde permettant le clonage de l'ADNc complet contenant la phase de lecture ouverte complète codant pour le gène correspondant.

Les séquences SEQ. ID. NO. 2 et SEQ. ID. NO. 3 sont les suivantes :

- SEQ. ID. NO. 2 : 5'-CTG ACC TTT CAG TTT TCC ATA GC-3'

- SEQ. ID. NO. 3 : 5'-ATC TAT TTC TGC CAA ACA AGA A-3'

Dans un premier temps, une réaction de polymérisation en chaîne (PCR) est réalisée sur une série de banques d'ADNc disponibles commercialement en utilisant les amorces de séquences SEQ. ID. NO. 2 et SEQ. ID. NO. 3. Les conditions de réaction incluent 0,5 μM de Fwdl (SEQ. ID. NO. 2) et de Revl (SEQ. ID. NO. 3), 200 μM de déoxynucléotides triphosphate (ou dNTPs : dATP, dCTP, dGTP et dTTP), 10 mM Tris-HCl (pH 8,3), 50 mM KCl, 1,5 mM $MgCl_2$ et 0,5 U Taq ADN polymérase dans un volume final de 50 μl. Les paramètres de cycles thermiques comprennent une dénaturation à 94° C pendant 30 s, une hybridation des amorces à 60° C pendant 1 minute et une extension de polymérisation à 72° C pendant 30 s (avec une extension finale de 5 minutes).

Les produits obtenus par la PCR sont séparés sur gel d'agarose 1 % et visualisés à l'aide d'une coloration au bromure d'éthidium. Les résultats montrent une bande de 245 paires de bases dans les banque d'ADNc de cerveau, d'hypophyse, de poumon, de rein et d'intestin humain (Quantum) et 290 paires de bases dans une banque d'ADNc de sein et de prostate humain. Ces résultats sont reproduits en **Figure 1.**

Les produits ainsi obtenus par la PCR sont purifiés par électro-élution et sont utilisés comme sonde pour le clonage de l'ADNc contenant la phase de lecture ouverte complète en utilisant les recommandations du fabricant pour l'utilisation du kit de clonage d'ADNc ClonCapture (Clontech).

Les séquences en acides nucléiques des clones positifs sont déterminées à l'aide d'un séquenceur automatique. Il s'agit des séquences SEQ. ID. NO. 4 et SEQ. ID. NO. 5 reproduites ci-après :

- SEQ. ID. NO. 4 :

```
  1 aggagggtct gaaggtggga gggcagctcc atgagcagcc actgttcctg acagccgaa
 61 gtgccaggca gggggaggtt tccatgtcca ggagggagag gacctccacc ctctgacctt
121 tcagttttcc atagcagccc agtgtgaagc tcggcggggg tgggcagggc atcaggacaa
181 gatgaagatg ctggctctgg aatgaggact ccccttgggc taaggggtag tggctggcag
241 aggttgccca gctcttttgg aggccacagg gaatagaggt ggggtggtgc ttccgttcct
301 gtgaggcccg cccccctgag ccccctcgtc tgcttcttgt ttggcagaaa tagatgtggg
361 gcacagcccc tagatgatgg gaatctgccc aagctgtgtc ctctggggaa tgaaatcttg
421 ctcctcatgc agcttcccct ttcccttgcg gggtgggggc tgggggtgcc aaggctccac
481 ccttggaggg ggttgctggg ggtgccaagg ctccaccctt tgctcgctgg gcagagggaa
541 ggaagcacct ccctgtatgc tctgcagggg cccctgcagg ccactttgcc tcagaggaag
601 gaggagaggt cccctgggaa aatgcgcaca cacacacaca cacacacacg cacacacaca
661 cgcacatgca cacacgcacg catgcacaca cacacacata tgtgcagggg tgcagcctaa
```

```
721 gtcagtggag ccaggactgg ctgcgcagct gggcctgccc cattcttgga gtccccaggg
781 aatgagcctc aggatccact tcgtcccttg tttctaagcc aggctgcacg gccgttcctg
841 ggtgtgaaag cattctgtcc tgtctccagt cagagggaac cgcccccaaa ctagaaagct
901 aatttccccc gaaggttcat gaagccagag gcatgccctt gggggttggt cgggagaagg
961 gtgagaggca gtgctgtgga aggggcctgg cccctgctgc ctgcgattc
```

- SEQ. ID. NO. 5 :

```
   1 aggagggtct gaaggtggga gggcagctcc atgagcagcc actgttcctg accagccgaa
  61 gtgccaggca gggggaggtt tccatgtcca ggagggagag gacctccacc ctctgacctt
 121 tcagttttcc atagcagccc agtgtgaagc tcggcggggg tgggcagggc atcaggacaa
 181 gatgaagatg ctggctctgg aatgaggact ccccttgggc taaggggtag tggctggcag
 241 aggttgccca gctcttttgg aggccacagg gaatagaggt ggggtggcaa caggaaatag
 301 ggtgtgggct gtagaccata gtgggtgggg tggtgcttcc gttcctgtga ggcccgcccc
 361 cctgagcccc ctcgtctgct tcttgtttgg cagaaataga tgtggggcac agcccctaga
 421 tgatgggaat ctgcccaagc tgtgtcctct ggggaatgaa atcttgctcc tcatgcagct
 481 tccccttcc cttgcggggt gggggctggg ggtgccaagg ctccaccctt ggaggggggtt
 541 gctgggggtg ccaaggctcc accctttgct cgctgggcag agggaaggaa gcacctccct
 601 gtatgctctg caggggcccc tgcaggccac tttgcctcag aggaaggagg agaggtcccc
 661 tgggaaaatg cgcacacaca cacacacaca cacacgcaca cacacacgca catgcacaca
 721 cgcacgcatg cacacacaca cacatatgtg caggggtgca gcctaagtca gtggagccag
 781 gactggctgc gcagctgggc ctgccccatt cttggagtcc ccagggaatg agcctcagga
 841 tccacttcgt cccttgtttc taagccaggc tgcacggccg ttcctgggtg tgaaagcatt
 901 ctgtcctgtc tccagtcaga gggaaccgcc cccaaactag aaagctaatt tcccccgaag
 961 gttcatgaag ccagaggcat gcccttgggg gttggtcggg agaagggtga gaggcagtgc
1021 tgtggaaggg gcctggcccc tgctgcctgc gattc
```

Exemple 2 : clonage dans un vecteur d'expression de SEQ. ID. NO. 4 et de SEQ. ID.NO.5

Les séquences d'acides nucléiques SEQ. ID. NO. 4 et SEQ. ID. NO. 5 donnent lieu à la synthèse de nouvelles amorces en 5' F1 et en 3' R1 de séquences respectives SEQ. ID. NO. 6 et SEQ. ID. NO. 7 (reproduites ci-après). Ces séquences contiennent respectivement les sites de restriction HindIII et EcoRI.

Les séquences SEQ. ID. NO. 6 et SEQ. ID. NO. 7 sont les suivantes :

- SEQ. ID. NO. 6 : 5'- GCA AGC TTG CGG GGG AGG AGG AGG G -3'
- SEQ. ID. NO. 7 : 5'- CGG AAT TCC CGG GGG GAA TCG CAG -3'

Une réaction PCR est réalisée en utilisant les mêmes conditions de réaction que celles décrites dans l'exemple 1 avec les amorces SEQ. ID. NO. 6 et SEQ. ID. NO. 7. Les produits obtenus par ladite réaction PCR peuvent être insérés directement dans un vecteur d'expression de type mais de manière non exclusive pCDNA3.1 (In Vitrogen). Après l'étape d'amplification par PCR, les produits sont soumis à une hydrolyse HindIII et EcoRI afin de libérer des extrémités contenant ces séquences puis purifiés à partir du gel d'agarose par électroélution.

Les séquences SEQ. ID. NO. 4 et SEQ. ID. NO. 5 sont ensuite insérées dans un vecteur d'expression comme par exemple pCDNA3.1 (In Vitrogen). L'analyse par digestion par des enzymes de restriction convenables de différents clones bactériens ayant reçu ledit vecteur d'expression permet d'isoler les clones bactériens contenant la nouvelle construction « vecteur d'expression/SEQ ID NO 4 » ou « vecteur d'expression/SEQ ID NO 5 ».

La préparation en grande quantité de ces constructions plasmidiques est réalisée à l'aide d'un kit de purification d'ADN plasmidique (Qiagen).

**Propriétés des polynucléotides de séquences SEO. ID. NO. 4 et SEO. ID. NO. 5**

Expression des polynucléotides de séquences SEQ. ID. NO. 4 et de SEQ. ID. NO. 5 dans des tissus tumoraux humains

*Préparation des ARNs à partir de cultures cellulaires et de tissus tumoraux*

Les cellules en culture ou les tissus tumoraux sont conservés à -80° C avant les étapes d'extraction des ARNs totaux. L'extraction des ARNs totaux est basée sur une technique décrite dans la littérature scientifique (Chomczynski et Sacchi, *Anal. Biochem.* (1987), **162,** 156) à l'aide du réactif Trizol (Gibco/BRL). La qualité des ARNs ainsi extraits est analysée sur gel d'agarose 1 % en présence de Bromure d'éthidium.

*Transcription inverse à partir des ARN*

Les ARNs totaux sont transcrits de manière inverse avec des amorces Oligo(dT) en utilisant la transcriptase inverse Superscript® comme suggéré dans le manuel du fabricant (Gibco/BRL).

*Réaction de polymérisation en chaîne (PCR) sur les produits obtenus à partir de la transcription inverse*

L'analyse de l'expression des séquences SEQ. ID. NO. 4 ou SEQ. ID. NO. 5 est réalisée par réaction de polymérisation en chaine (PCR) sur les produits de la transcription inverse en utilisant les amorces en 5' Fwdl et en 3' Revl de séquences respectives SEQ. ID. NO. 2 et SEQ. ID. NO. 3. Les conditions de réaction incluent 0,5 μM de Fwdl et de Rev1, 200 μM dNTPs, 10 mM Tris-HCl (pH 8,3), 50 mM KCl, 1,5 mM $MgCl_2$ et 0,5 U Taq ADN polymérase dans un volume final de 50 μl. Les paramètres de cycles thermiques comprennent une dénaturation à 94° C pendant 30 s, une hybridation des amorces à 60° C pendant 1 minute et une extension de polymérisation à 72° C pendant 30 s (avec une extension finale de 5 minutes).

Les produits obtenus par la PCR sont séparés sur gel d'agarose 1 % et visualisés à l'aide d'une coloration au bromure d'éthidium.

Dans certains cas, une analyse d'hybridation sur membrane de nylon a été réalisée en utilisant comme sonde la séquence SEQ. ID. NO. 4 marquée avec du alphaP32-dCTP en utilisant un kit de marquage aléatoire (Pharmacia). La membrane est ensuite lavée dans des conditions de stringence faible (2 XSSC, 0,1 % SDS à 60° C) puis dans des conditions de stringence forte (0,1 XSSC, 0,1 % SDS à 65° C). La membrane est ensuite exposée à un film Kodac XAR.

*Analyse de l'expression des polynucléotides de séquences SEQ. ID. NO. 4 et SEQ. ID. NO. 5 dans des échantillons de tumeurs du sein*

L'analyse de l'expression des polynucléotides de séquences SEQ. ID. NO. 4 et SEQ. ID. NO. 5 a été réalisée sur 15 échantillons tumoraux extraits par chirurgie de patientes atteintes de tumeurs du sein.

Les ADNc obtenus selon le protocole décrit ci-dessus ont été normalisés à l'aide d'un marqueur exprimé de manière stable G3PDH (Glycéraldéhyde 3-phosphate déhydrogénase) afin de contrôler les variations de quantité d'ARNs ou d'ADNc entre les échantillons. Les produits de la réaction PCR ont ensuite été hybridés sur une membrane de nylon selon le protocole décrit ci-dessus.

Les résultats obtenus, représentés en **Figure 2**, montrent que, par cette approche, la présence des polynucléotides de séquences SEQ. ID. NO. 4 et SEQ. ID. NO. 5 peut être visualisée dans des échantillons tumoraux de carcinomes mammaires.

Inhibition de la prolifération cellulaire par les polynucléotides de séquences SEQ. ID. NO. 4 et de SEQ. ID. NO. 5

Des cellules de tumeur de la prostate DU 145 (Code ATCC) sont cultivées dans du milieu DMEM (milieu de Eagle modifié par Dulbeco) contenant 100 U/ml de pénicilline et 100 μg/ml de sulfate de streptomycine, complémentée avec du sérum de veau foetal à 10 %. Les cellules sont sous-cultivées 24 h avant le protocole de transfection permettant un métabolisme normal des cellules et une meilleure efficacité de transfection. Des concentrations croissantes (1, 5 et 10 μg) de vecteur seul (pCDNA3.1) ou de vecteur contenant le polynucléotide de séquence SEQ. ID. NO. 4 (1, 5 et 10 μg) ou le polynucléotide de séquence SEQ. ID. NO. 5 (1, 5 et 10 μg) ont été réalisées en utilisant le réactif Effectene®

selon les recommandations du fabricant (Qiagen).

Les cellules sont récupérées 48 h après la transfection par un traitement à la trypsine puis comptées. Dans le même temps, les cellules sont centrifugées à 5000 tours/min puis congelées à -80° C pour une extraction d'ARNs comme décrit plus haut (cf. partie intitulée « *Préparation des ARNs à partir de cultures cellulaires et de tissus tumoraux* »).

Les résultats sont reproduits en **Figure 3** (polynucléotide de séquence SEQ. ID. NO. 4) et **Figure 4** (polynucléotide de séquence SEQ. ID. NO. 5). Ils indiquent que l'ADNc codant pour le polynucléotide de séquence SEQ. ID. NO. 4 ou celui codant pour le polynucléotide de séquence SEQ. ID. NO. 5 est exprimé de manière proportionnelle à la quantité de vecteur d'expression contenant le polynucléotide de séquence SEQ. ID. NO. 4 ou le polynucléotide de séquence SEQ. ID. NO. 5 (Partie A). De plus, les résultats indiquent que la croissance des cellules est inhibée de manière significative en présence de concentrations croissantes de vecteur d'expression contenant le polynucléotide de séquence SEQ. ID. NO. 4 ou le polynucléotide de séquence SEQ. ID. NO. 5 par rapport à la croissance des cellules transfectées avec 10 µg du vecteur seul (Partie B). La corrélation entre l'inhibition de la croissance des cellules tumorales et la quantité de vecteur transfecté contenant le polynucléotide de séquence SEQ. ID. NO. 4 ou le polynucléotide de séquence SEQ. ID. NO. 5 est égale à 1 ou à 0,96 respectivement.

<u>**Polypeptide codé à partir d'un fragment de la séquence SEO. ID. NO. 5**</u>

Dans la séquence SEQ. ID. NO. 5, l'on observe une phase ouverte de lecture avec la présence d'un codon initiateur (ATG) codant pour une méthionine initiatrice en position 420 et d'un codon stop (UAA) en position 861. Au polynucléotide ainsi traduit correspond une protéine de séquence SEQ. ID. NO. 8, composée de 147 acides aminés et reproduite ci-après :

```
M M G I C P S C V L W G M K S C S S C S F P F P L R G G G W   30
G C Q G S T L G G G C W G C Q G S T L C S L G R G K E A P P   60
C M L C R G P C R P L C L R G R R R G P L G K C A H T H T H   90
T H A H T H A H A H T H A C T H T H I C A G V Q P K S V E P   120
G L A A Q L G L P H S W S P Q G M S L R I H F V P C F         147
```

Le polynucléotide auquel correspond la protéine de séquence SEQ. ID. NO. 8 possède une séquence nucléotidique SEQ. ID. NO. 9 reproduite ci-après :

```
  1 atgatgggaa tctgcccaag ctgtgtcctc tggggaatga aatcttgctc ctcatgcagc
 61 ttcccctttc ccttgcgggg tgggggctgg gggtgccaag gctccaccct tggagggggt
121 tgctgggggt gccaaggctc caccctttgc tcgctgggca gagggaagga agcacctccc
181 tgtatgctct gcaggggccc ctgcaggcca ctttgcctca gaggaaggag gagaggtccc
241 ctgggaaaat gcgcacacac acacacacac acacacgcac acacacacgc acatgcacac
301 acgcacgcat gcacacacac acacatatgt gcaggggtgc agcctaagtc agtggagcca
361 ggactggctg cgcagctggg cctgccccat tcttggagtc cccagggaat gagcctcagg
421 atccacttcg tcccttgttt ctaa
```

<u>**Légende des figures**</u>

La Figure 1 représente les résultats obtenus par PCR après séparation sur gel d'agarose à 1 % et visualisation à l'aide d'une coloration au bromure d'éthidium.

La **Figure 2** représente les résultats obtenus par Southern blot sur 15 échantillons de carcinomes mammaires (nommés de S1 à S15).

La **Figure 3** montre, dans sa partie A, l'expression de l'ADNc codant pour le polynucléotide de séquence SEQ. ID. NO. 4 dans des cellules DU 145 (tumeur de la prostate), et dans sa partie B, l'inhibition de la croissance des cellules en présence de concentrations croissantes de vecteur d'expression contenant le polynucléotide de séquence SEQ. ID. NO. 4.

Enfin, la **Figure 4** montre, dans sa partie A, l'expression de l'ADNc codant pour le polynucléotide de séquence SEQ. ID. NO. 5 dans des cellules DU 145 (tumeur de la prostate), et dans sa partie B, l'inhibition de la croissance des cellules en présence de concentrations croissantes de vecteur d'expression contenant le polynucléotide de séquence SEQ. ID. NO. 5.

## Revendications

1. Polynucléotide isolé comprenant la séquence polynucléotidique SEQ. ID. NO. 5.

2. Polynucléotide anti-sens comprenant la séquence complémentaire à celle du polynucléotide selon la revendication 1.

3. Polynucléotide isolé de séquence nucléotidique SEQ. ID. NO. 9

4. Polynucléotide isolé de séquence nucléotidique complémentaire à la séquence nucléotidique SEQ. ID. NO. 9.

5. Polypeptide isolé de séquence SEQ. ID. NO. 8.

6. Anticorps monoclonal, ou fragment de liaison de l'antigène de celui-ci, qui fixe spécifiquement un polypeptide isolé selon la revendication 5.

7. Vecteur d'expression comprenant un polynucléotide isolé de séquence polynucléotidique SEQ. ID. NO. 5.

8. Cellule hôte transformée ou transfectée avec un vecteur d'expression selon la revendication 7.

9. Polypeptide isolé selon la revendication 5 pour l'utilisation comme médicament.

10. Composition pharmaceutique comprenant un polypeptide isolé de séquence SEQ. ID. NO. 8 avec un ou des excipients pharmaceutiquement acceptables.

11. Utilisation d'un polypeptide isolé de séquence SEQ ID N°8 pour préparer un médicament destiné à prévenir ou traiter un cancer.

## Claims

1. Isolated polynucleotide comprising the polynucleotide sequence SEQ.ID.NO.5.

2. Anti-sense polynucleotide comprising the complementary sequence to that of the polynucleotide according to claim 1.

3. Isolated polynucleotide of the polynucleotide sequence SEQ.ID.N0.9.

4. Isolated polynucleotide of the complementary polynucleotide sequence to that of the polynucleotide sequence SEQ.ID.NO.9.

5. Isolated polypeptide of the sequence SEQ.ID.NO.8.

6. Monoclonal antibody, or a fragment of an antigen bond of the latter, which specifically binds an isolated polypeptide according to claim 5.

7. Expression vector comprising an isolated polynucleotide of the polynucleotide sequence SEQ.ID.N0.5.

8. Host cell transformed or transfected with an expression vector according to claim 7.

9. Isolated polypeptide according to claim 5 for a use as a medicament.

10. Pharmaceutical composition comprising an isolated polypeptide of the sequence SEQ.ID.NO.8 with one or more pharmaceutically acceptable excipients.

11. Use of an isolated polypeptide of sequence SEQ.ID.NO.8 for preparing a medicament intended to prevent or treat cancer.

## Patentansprüche

1. Isoliertes Polynucleotid, das die Polynucleotidsequenz SEQ.ID.Nr.5 umfasst.

2. Antisense-Polynucleotid, das die zu der des Polynucleotids nach Anspruch 1 komplementäre Sequenz umfasst.

3. Isoliertes Polynucleotid mit der Nucleotidsequenz SEQ. ID. Nr. 9.

4. Isoliertes Polynucleotid mit der zur Nucleotidsequenz SEQ.ID.Nr.9 komplementären Nucleotidsequenz.

5. Isoliertes Polypeptid mit der Sequenz SEQ.ID.Nr.8.

6. Monoklonaler Antikörper oder dessen Fragment zur Bindung des Antigens, der bzw. das ein isoliertes Polypeptid nach Anspruch 5 spezifisch bindet.

7. Expressionsvektor, umfassend ein isoliertes Polynucleotid mit der Polynucleotidsequenz SEQ.ID.Nr.5.

8. Wirtszelle, transformiert oder transfektiert von einem Expressionsvektor nach Anspruch 7.

9. Isoliertes Polypeptid nach Anspruch 5 für die Verwendung als Medikament.

10. Pharmazeutische Zusammensetzung, umfassend ein isoliertes Polypeptid mit der Sequenz SEQ.ID.Nr.8 mit einem oder mehreren pharmazeutisch annehmbaren Hilfsstoffen.

11. Verwendung eines isolierten Polypeptids mit der Sequenz SEQ.ID.Nr.8 für die Herstellung eines Medikaments, das für die Prävention oder Behandlung von Krebs bestimmt ist.

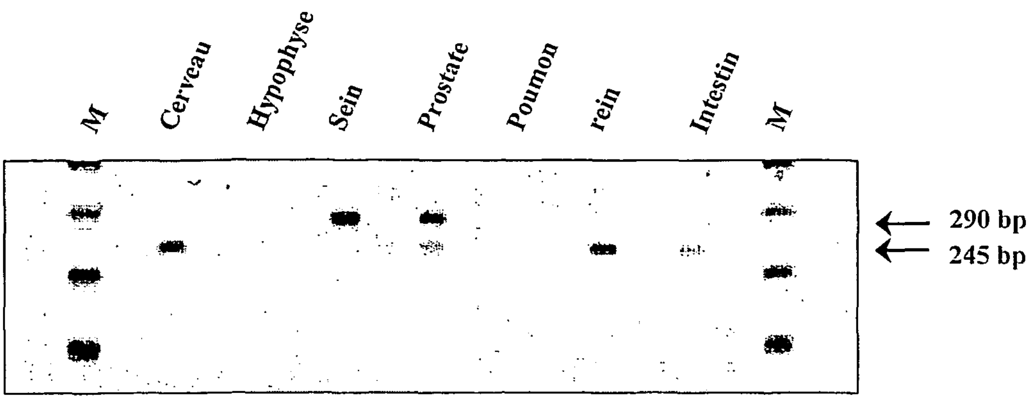


**Figure 1**

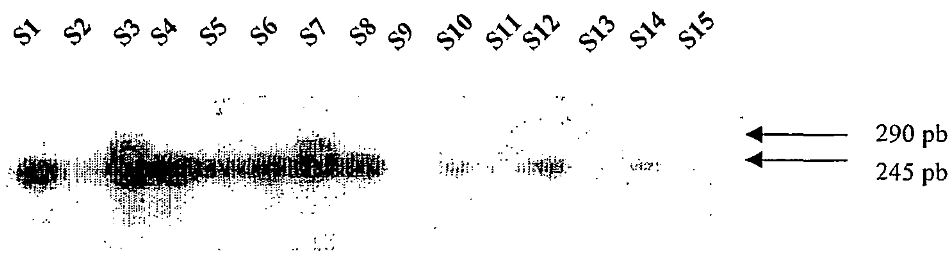


**Figure 2**

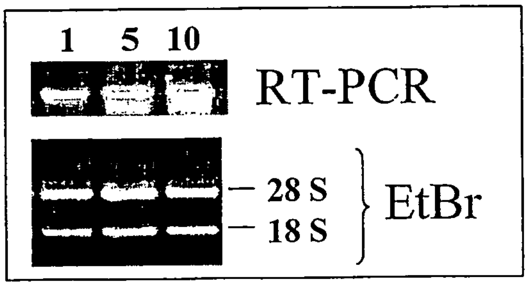
1
5
10
RT-PCR
28 S
18 S
EtBr

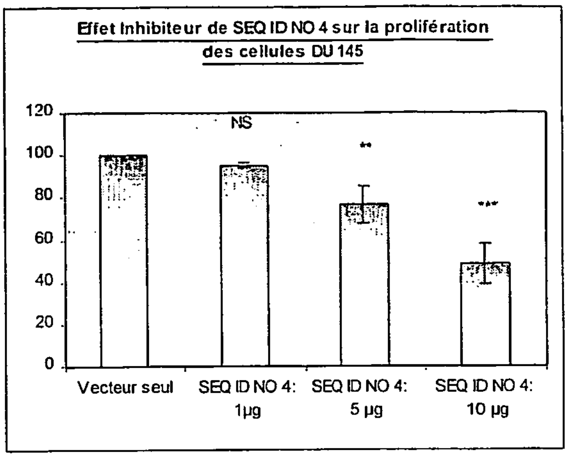
Effet Inhibiteur de SEQ ID NO 4 sur la prolifération des cellules DU 145
120
100
80
60
40
20
0
NS
Vecteur seul
SEQ ID NO 4: 1µg
SEQ ID NO 4: 5 µg
SEQ ID NO 4: 10 µg

A

B

**Figure 3**

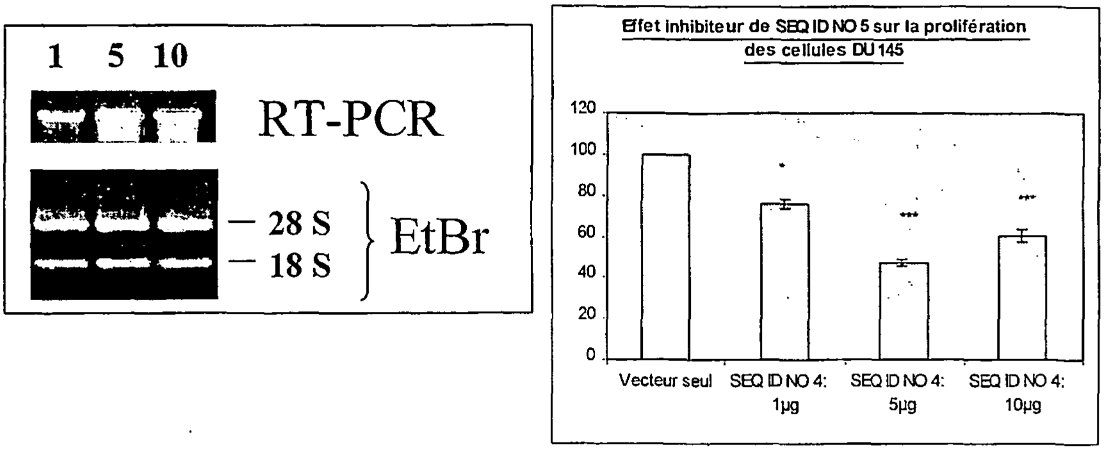
1 5 10
RT-PCR
28 S
18 S
EtBr
Effet inhibiteur de SEQ ID NO 5 sur la prolifération des cellules DU145
120
100
80
60
40
20
0
*
***
***
Vecteur seul
SEQ ID NO 4: 1µg
SEQ ID NO 4: 5µg
SEQ ID NO 4: 10µg

A B

**Figure 4**